# NEW EUROPEAN PATENT SPECIFICATION

(11) **EP 1 317 545 B2**
(45) Date of publication and mention of the opposition decision: **28.10.2009**
(45) Mention of the grant of the patent: 29.03.2006
(21) Application number: 01976189.9
(22) Date of filing: 05.09.2001
(51) Int. Cl.: C12N 15/31, C12P 13/04, C12P 13/08, C12Q 1/68

(54) **Coryneform bacteria transformed with sequences encoding the PKND gene and their use in producing L-amino acids**
Coryneforme Bakteriën transformiert mit dem PKND-Gen kodierenden Nukleotidsequenzen und deren Verwendung zur Herstellung von L -Aminosäuren
Bactéries coryneformes transformées avec des séquences nucléotidiques codant pour le gene PKND et leur utilisation dans la préparation d'acides aminés L

(30) Priority: 12.09.2000 DE 10044948; 25.04.2001 DE 10120094
(43) Date of publication of application: 11.06.2003
(73) Proprietor: Evonik Degussa GmbH, 45128 Essen (DE)
(72) Inventor: BATHE, Brigitte, 33154 Salzkotten (DE); SCHRÖDER, Indra, 33803 Steinhagen (DE); FARWICK, Mike, 33615 Bielefeld (DE); HERMANN, Thomas, 33739 Bielefeld (DE)
(86) International application number: PCT/EP2001/010210
(87) International publication number: WO 2002/022632

(56) References cited:
- EP-A- 1 108 790
- WO-A-00/17379
- US-A- 4 411 997
- DATABASE EMBL [Online] MTV021 Accession Number AL021957, 23 February 1998 (1998-02-23) COLE ET AL. : "Mycobacterium tuberculosis H37Rv complete genome; segment 97/162" XP002191212
- AV-GAY Y., AND EVERETT M.: "The eukaryotic-like Ser/thr protein kinases of Mycobacterium tuberculosis" TRENDS IN MICROBIOLOGY, vol. 8, no. 5, May 2000 (2000-05), pages 238-244, XP002191211
- SCHRUMPF B ET AL: "A FUNCTIONALLY SPLIT PATHWAY FOR LYSINE SYNTHESIS IN CORYNEBACTERIUM GLUTAMICUM" JOURNAL OF BACTERIOLOGY, WASHINGTON, DC, US, vol. 173, no. 14, July 1991 (1991-07), pages 4510-4516, XP000884180 ISSN: 0021-9193
- DATABASE EMBL [Online] Accession number; AX127150, nt 202141-205620, 10 May 2001 (2001-05-10) NAKAGAWA ET AL.: "Sequence 7066 from Patent EP1108790" XP002191244
- DATABASE EMBL [Online] Accession number AX125968, 10 May 2001 (2001-05-10) NAKAGAWA ET AL.: "Sequence 5884 from Patent EP1108790" XP002191701
- RYKX A. ET AL: 'Protein kinase D: a family affair' FEBS LETTERS vol. 546, no. 1, 12 May 2003, AMSTERDAM, NL, pages 81 - 86

## Description

### Field of the Invention

The invention provides transformed coryneform bacteria and a fermentation process for the preparation of amino acids using bacteria in which the endogenous pknD gene is overexpressed.

### Prior Art

L-Amino acids, especially L-lysine, are used in human medicine, in the pharmaceutical industry, in the food industry and very especially in animal nutrition.

It is known that amino acids are prepared by the fermentation of strains of corynebacteria, especially Corynebacterium glutamicum. Because of their great importance, attempts are constantly being made to improve the preparative processes. Improvements to the processes may relate to measures involving the fermentation technology, e.g. stirring and oxygen supply, or the composition of the nutrient media, e.g. the sugar concentration during fermentation, or the work-up to the product form, e.g. by ion exchange chromatography, or the intrinsic productivity characteristics of the microorganism itself.

The productivity characteristics of these microorganisms are improved by using methods of mutagenesis, selection .and mutant choice to give strains which are resistant to antimetabolites or auxotrophic for metabolites important in regulation, and produce amino acids.

Methods of recombinant DNA technology have also been used for some years to improve L-amino acid-producing strains of Corynebacterium by amplifying individual amino acid biosynthesis genes and studying the effect on amino acid production.

### Object of the Invention

The object which the inventors set themselves was to provide novel measures for improving the preparation of amino acids by fermentation.

### Summary of the Invention

When L-amino acids or amino acids are mentioned hereafter, they are understood as meaning one or more amino acids, including their salts, selected from the group comprising L-asparagine, L-threonine, L-serine, L-glutamate, L-glycine, L-alanine, L-cysteine, L-valine, L-methionine, L-isoleucine, L-leucine, L-tyrosine, L-phenylalanine, L-histidine, L-lysine, L-tryptophan and L-arginine. L-Lysine is particularly preferred.

When L-lysine or lysine is mentioned hereafter, it is understood as meaning not only the bases but also the salts, e.g. lysine monohydrochloride or lysine sulfate.

The invention provides transformed coryneform bacteria, wherein a polynucleotide from coryneform bacterium encoding a polypeptide having an amino acid sequence which is at least 90 % identical to the amino acid sequence set out in SEQ ID NO: 2 or having an amino acid sequence of the variant of SEQ ID . NO:2 with amino acid exchanges in the section between position 661 and 669 are overexpressed. Said amino acid exchanges are selected from the group:
a) SEQ ID NO:2 wherein the glutamic acid at position 664 is exchanged; or
b) SEQ ID NO:2 wherein the glycine at position 666 is exchanged; or
c) SEQ ID NO:2 wherein the glutamic acid at position 664 and glycine at position 666 are exchanged.

Prefered are bacteria wherein
a) the glutamic acid at position 664 of SEQ ID NO: 2 is replaced by L-lysine or L-arginine.
b) the glycine at position 666 of SEQ ID NO:2 is replaced by L-serine or L-threonine, and
c) the glutamic acid at position 664 is replaced by L-lysine and the glycine at position 666 of SEQ ID NO:2 is replaced by L-serine (SEQ ID NO:4).

Preferred are also bacteria, wherein the polynucleotide is chosen from the group:
a) nucleotide sequences as set out in SEQ ID NO:1 nucleotides 512 to 2731 or SEQ ID NO:3 nucleotides 512 to 2731; or
b) nucleotide sequences as set out in SEQ ID NO:1 or SEQ ID NO:3; or
c) nucleotide sequence corresponding to nucleotides. 512 to 2731 of sequence SEQ ID NO:1 or nucleotides 512 to 2731 of sequence SEQ ID NO:3 within the degeneracy of the genetic code; or
d) nucleotide sequences with neutral sense mutations in SEQ ID NO:1 nucleotides 512 to 2731 or SEQ ID NO:3. nucleotides 512 to 2731.
According to the invention overexpression is achieved by a method chosen from the group:
a) increasing the copy number of said polynucleotide, or
b) using a promoter; or
c) introducing said polynucleotide into the chromosome:

The invention also provides process for the preparation of L-amino acids, comprising
a) fermenting of said bacteria,
b) enrichment of the L-amino acid in the medium or in the cells of the bacteria.

The description also provides:
a replicable polynucleotide, especially DNA, containing the nucleotide sequence as shown in SEQ ID No. 1,
a polynucleotide coding for a polypeptide containing the amino acid sequence as shown in SEQ ID No. 2,
a vector containing the polynucleotide according to the invention, especially a shuttle vector or plasmid vector, and
corynebacteria which contain the vector or in which the endogenous pknD gene is overexpressed.

The description also provides polynucleotides consisting substantially of a polynucleotide sequence which are obtainable by screening, by means of hybridization, of an appropriate gene library of a Corynebacterium, containing the complete gene or parts thereof, with a probe containing the sequence of the polynucleotide of the invention according to SEQ ID No. 1 or a fragment thereof, and by isolation of said polynucleotide sequence.

### Detailed Description of the Invention

As hybridization probes for RNA, cDNA and DNA, polynucleotides containing the sequences according to the description are suitable for isolating the full length of nucleic acids, or polynucleotides or genes, coding for protein kinase D, or for isolating nucleic acids, or polynucleotides or genes, whose sequence exhibits a high degree of similarity to the sequence of the pknD gene. They are also suitable for incorporation into so-called arrays, micro-arrays or DNA chips for detecting and determining the corresponding polynucleotides.

Polynucleotides containing said sequences are further suitable as primers for the preparation, by the polymerase chain reaction (PCR), of DNA of genes coding for protein kinase D.

Such oligonucleotides serving as probes or primers contain at least 25, 26, 27, 28, 29 or 30, preferably at least 20., 21, 22, 23 or 24 and very particularly preferably at least 15, 16, 17, 18 or 19 consecutive nucleotides.
Oligonucleotides with a length of at least 31, 32, 33, 34, 35, 36, 37, 38, 39 or 40 or at least 41, 42, 43, 44, 45, 46, 47, 48, 49 or 50 nucleotides are also suitable. Oligonucleotides with a length of at least 100, 150, 200, 250 or 300 nucleotides may also be suitable.

"Isolated" means separated from its natural environment.

" Polynucleotide" refers in general to polyribonucleotides and polydeoxyribonucleotides, it being possible for the RNAs or DNAs in question to be unmodified or modified.

The described polynucleotides include a polynucleotide according to SEQ ID No. 1 or a fragment prepared therefrom, as well as polynucleotides which are in particular at least 70% to 80%, preferably at least 81% to 85%, particularly preferably at least 86% to 90% and very particularly preferably at least 91%, 93%, 95%, 97% or 99% identical to the polynucleotide according to SEQ ID No.1 or a fragment prepared therefrom.

"Polypeptides" are understood as meaning peptides or proteins containing two or more amino acids bonded via peptide links.

The described polypeptides include a polypeptide according to SEQ ID No. 2, with the biological activity of protein kinase D and also those which are at least 70% to 80%, preferably at least 81% to 85%, particularly preferably at least 86% to 90% and very particularly preferably at least 91%, 93%, 95%, 97% or 99% identical to the polypeptide according to SEQ ID No. 2, and have said activity.

The invention further relates to a fermentation process for the preparation of amino acids selected from the group comprising L-asparagine, L-threonine, L-serine, L-glutamate, L-glycine, L-alanine, L-cysteine, L-valine, L-methionine, L-isoleucine, L-leucine, L-tyrosine, L-phenylalanine, L-histidine, L-lysine, L-tryptophan and L-arginine, using corynebacteria which, in particular, already produce amino acids and in which the nucleotide sequences coding for the pknD gene are overexpressed.

In this context the term "amplification" describes the increase in the intracellular activity, in a microorganism, of one or more enzymes which are coded for by the appropriate DNA, for example by increasing the copy number of the gene(s) or allele(s), using a strong promoter or using a gene or allele coding for an appropriate enzyme with a high activity, and optionally combining these measures.

By over-expression the activity or concentration of the corresponding protein is in general increased by at least 10%, 25%, 50%, 75%, 100%, 150%, 200%, 300%, 400% or 500%, up to a maximum of 1000% or 2000%, based on that of the wild-type protein or the activity or concentration of the protein in the starting microorganism.

The microorganisms provided by the present invention can produce L-amino acids from glucose, sucrose, lactose, fructose, maltose, molasses, starch or cellulose or from glycerol and ethanol. Said microorganisms can be representatives of corynebacteria, especially of the genus Corynebacterium. The species Corynebacterium glutamicum may be mentioned in particular in the genus Corynebacterium, being known to those skilled in the art for its ability to produce L-amino acids.

The following known wild-type strains:
Corynebacterium glutamicum ATCC13032
Corynebacterium acetoglutamicum ATCC15806
Corynebacterium acetoacidophilum ATCC13870
Corynebacterium thermoaminogenes FERM BP-1539
Corynebacterium melassecola ATCC17965
Brevibacterium flavum ATCC14067
Brevibacterium lactofermenturn ATCC13869, and
Brevibacterium divaricatum ATCC14020
and L-amino acid-producing mutants or strains prepared therefrom, are particularly suitable strains of the genus Corynebacterium, especially of the species Corynebacterium glutamicum (C. glutamicum).

The pknD gene of C. glutamicum coding for the enzyme protein kinase D (EC 2.7.1.37) has been isolated.

The first step in isolating the pknD gene or other genes of C. glutamicum is to construct a gene library of this microorganism in Escherichia coli (E. coli). The construction of gene libraries is documented in generally well-known textbooks and manuals. Examples which may be mentioned are the textbook by Winnacker entitled From Genes to Clones, Introduction to Gene Technology (Verlag Chemie, Weinheim, Germany, 1990) or the manual by Sambrook et al. entitled Molecular Cloning, A Laboratory Manual (Cold Spring Harbor Laboratory Press, 1989). A very well-known gene library is that of the E. coli K-12 strain W3110, which was constructed by Kohara et al. (Cell 50, 495-508 (1987)) in λ vectors. Bathe et al. (Molecular and General Genetics 252, 255-265, 1996) describe a gene library of C. glutamicum ATCC13032, which was constructed using cosmid vector SuperCos I (Wahl et al., 1987, Proceedings of the National Academy of Sciences USA 84, 2160-2164) in the E. coli K-12 strain NM554 (Raleigh et al., 1988, Nucleic Acids Research 16, 1563-1575).

Börmann et al. (Molecular Microbiology 6(3), 317-326 (1992)) in turn describe a gene library of C. glutamicum ATCC13032 using cosmid pHC79 (Hohn and Collins, Gene 11, 291-298 (1980)).

A gene library of C. glutamicum in E. coli can also be constructed using plasmids like pBR322 (Bolivar, Life Sciences 25, 807-818 (1979)) or pUC9 (Vieira et al., 1982, Gene 19, 259-268). Restriction- and recombination-defective E. coli strains are particularly suitable as hosts, an example being the strain DH5αmcr, which has been described by Grant et al. (Proceedings of the National Academy of Sciences USA 87 (1990) 4645-4649). The long DNA fragments cloned with the aid of cosmids can then in turn be subcloned into common vectors suitable for sequencing, and subsequently sequenced, e.g. as described by Sanger et al. (Proceedings of the National Academy of Sciences of the United States of America 74, 5463-5467, 1977).

The DNA sequences obtained can then be examined with known algorithms or sequence analysis programs, e.g. that of Staden (Nucleic Acids Research 14, 217-232 (1986)), that of Marck (Nucleic Acids Research 16, 1829-1836 (1988)) or the GCG program of Butler (Methods of Biochemical Analysis 39, 74-97 (1998)).

The DNA sequence of C. glutamicum coding for the pknD gene was found and, as SEQ ID No. 1, forms part of the present description. Furthermore, the amino acid sequence of the corresponding protein was derived from said DNA sequence by the methods described above. The resulting amino acid sequence of the pknD gene product is shown in SEQ ID No. 2.

Coding DNA sequences which result from SEQ ID No. 1 due to the degeneracy of the genetic code also form part of the invention. Likewise, DNA sequences which hybridize with SEQ ID No. 1 or portions of SEQ ID No. 1 form part of the invention. Furthermore, conservative amino acid exchanges, e.g. the exchange of glycine for alanine or of aspartic acid for glutamic acid in proteins, are known to those skilled in the art as "sense mutations", which do not cause a fundamental change in the activity of the protein, i.e. they are neutral. It is also known that changes at the N-and/or C-terminus of a protein do not substantially impair its function or may even stabilize it. Those skilled in the art will find information on this subject in Ben-Bassat et al. (Journal of Bacteriology 169, 751-757 (1987)), O'Regan et al. (Gene 77, 237-251 (1989)), Sahin-Toth et al. (Protein Sciences 3, 240-247 (1994)) and Hochuli et al. (Bio/Technology 6, 1321-1325 (1988)), inter alia, and in well-known textbooks on genetics and molecular biology.

Amino acid sequences which correspondingly result from SEQ ID No. 2 also form part of the descriptions.

Likewise, DNA sequences which hybridize with SEQ ID No. 1 or portions of SEQ ID No.1 form part of the description. Finally, DNA sequences which are prepared by the polymerase chain reaction (PCR) using primers resulting from SEQ ID No. 1 form part of the description. Such oligonucleotides typically have a length of at least 15 nucleotides.

Those skilled in the art will find instructions on the identification of DNA sequences by means of hybridization in inter alia the manual entitled " The DIG System User's Guide for Filter Hybridization" from Boehringer Mannheim GmbH (Mannheim, Germany, 1993) and in Liebl et al. (International Journal of Systematic Bacteriology (1991) 41, 255-260), inter alia. Hybridization takes place under stringent conditions; in other words, only hybrids for which the probe and the target sequence, i.e. the polynucleotides treated with the probe, are at least 70% identical are formed. It is known that the stringency of hybridization, including the washing steps, is influenced or determined by varying the buffer composition, the temperature and the salt concentration. The hybridization reaction is preferably carried out under relatively low stringency compared with the washing steps (Hybaid Hybridisation Guide, Hybaid Limited, Teddington, UK, 1996).

The hybridization reaction can be carried out for example using a 5x SSC buffer at a temperature of approx. 50°C - 68°C, it also being possible for probes to hybridize with polynucleotides which are less than 70% identical to the sequence of the probe. Such hybrids are less stable and are removed by washing under stringent conditions. This can be achieved for example by lowering the salt concentration to 2x SSC and subsequently to 0.5x SSC if necessary (The DIG System User's Guide for Filter Hybridization, Boehringer Mannheim, Mannheim, Germany, 1995), the temperature being adjusted to approx. 50°C - 68°C. It is possible to lower the salt concentration to 0.1x SSC if necessary. By raising the hybridization temperature in approx. 1 - 2°C steps from 50°C to 68°C, it is possible to isolate polynucleotide fragments which are e.g. at least 70%, at least 80% or at least 90% to 95% identical to the sequence of the probe used. Further instructions on hybridization are commercially available in the form of kits (e.g. DIG Easy Hyb from Roche Diagnostics GmbH, Mannheim, Germany, Catalog No. 1603558).

Those skilled in the art will find instructions on the amplification of DNA sequences with the aid of the polymerase chain reaction (PCR) in the manual by Gait entitled Oligonucleotide Synthesis: A Practical Approach (IRL Press, Oxford, UK, 1984) and in Newton and Graham: PCR (Spektrum Akademischer Verlag, Heidelberg, Germany, 1994), inter alia.

It has been found that, after overexpression of the pknD gene, the production of amino acids by corynebacteria is improved.

Overexpression can be achieved by increasing the copy number of the appropriate genes or mutating the promoter and regulatory region or the ribosome binding site located upstream from the structural gene. Expression cassettes incorporated upstream from the structural gene work in the same way. Inducible promoters additionally make it possible to increase the expression in the course of the production of amino acid by fermentation. Measures for prolonging the life of the mRNA also improve the expression. Furthermore, the enzyme activity is also enhanced by preventing the degradation of the enzyme protein. The genes or gene constructs can either be located in plasmids of variable copy number or integrated and amplified in the chromosome. Alternatively, it is also possible to achieve overexpression of the genes in question by changing the composition of the media and the culture technique.

Those skilled in the art will find relevant instructions in Martin et al. (Bio/Technology 5, 137-146 (1987)), Guerrero et al. (Gene 138, 35-41 (1994)), Tsuchiya and Morinaga (Bio/Technology 6, 428-430 (1988)), Eikmanns et al. (Gene 102, 93-98 (1991)), EP 0 472 869, US 4,601,893, Schwarzer and Pühler (Bio/Technology 9, 84-87 (1991)), Reinscheid et al. (Applied and Environmental Microbiology 60, 126-132 (1994)), LaBarre et al. (Journal of Bacteriology 175, 1001-1007 (1993)), WO 96/15246, Malumbres et al. (Gene 134, 15-24 (1993)), JP-A-10-229891, Jensen and Hammer (Biotechnology and Bioengineering 58, 191-195 (1998)) and Makrides (Microbiological Reviews 60, 512-538 (1996)), inter alia, and in well-known textbooks on genetics and molecular biology.

For amplification, the pknD gene according to the description has been overexpressed for example with the aid of episomal plasmids. Suitable plasmids are those which are replicated in corynebacteria. Numerous known plasmid vectors, e.g. pZ1 (Menkel et al., Applied and Environmental Microbiology (1989) 64, 549-554), pEKEx1 (Eikmanns et al., Gene 102, 93-98 (1991)) or pHS2-1 (Sonnen et al., Gene 107, 69-74 (1991)), are based on cryptic plasmids pHM1519, pBL1 or pGA1. Other plasmid vectors, e.g. those based on pCG4 (US-A-4,489,160), pNG2 (Serwold-Davis et al., FEMS Microbiology Letters 66, 119-124 (1990)1 or pAG1 (US-A-5,158,891), can be used in the same way.

Other suitable plasmid vectors are those which make it possible to use the gene amplification process by integration into the chromosome, as described for example by Reinscheid et al. (Applied and Environmental Microbiology 60, 126-132 (1994)) for the duplication or amplification of the hom-thrB operon. In this method the complete gene is cloned into a plasmid vector which can replicate in a host (typically E. coli), but not in C. glutamicum. Examples of suitable vectors are pSUP301 (Simon et al., Bio/Technology 1, 784-791 (1983)), pK18mob or pK19mob (Schäfer et al., Gene 145, 69-73 (1994)), pGEM-T (Promega Corporation, Madison, WI, USA), pCR2.1-TOPO (Shuman (1994), Journal of Biological Chemistry 269, 32678-84; US-A-5,487,993), pCR®Blunt (Invitrogen, Groningen, The Netherlands; Bernard et al., Journal of Molecular Biology 234, 534-541 (1993)), pEM1 (Schrumpf et al., 1991, Journal of Bacteriology 173, 4510-4516) or pBGS8 (Spratt et al., 1986, Gene 41, 337-342). The plasmid vector containing the gene to be amplified is then transferred to the desired strain of C. glutamicum by conjugation or transformation. The method of conjugation is described for example in Schafer et al. (Applied and Environmental Microbiology 60, 756-759 (1994)). Methods of transformation are described for example in Thierbach et al. (Applied Microbiology and Biotechnology 29, 356-362 (1988)), Dunican and Shivnan (Bio/Technology 7, 1067-1070 (1989)) and Tauch et al. (FEMS Microbiological Letters 123, 343-347 (1994)). After homologous recombination by means of a crossover event, the resulting strain contains at least two copies of the gene in question.

It has also been found that amino acid exchanges in the section between position 661 and position 669 of the amino acid sequence of protein kinase D, shown in SEQ ID No.2, improve the production of amino acids, especially lysine, by corynebacteria.

Preferably, L-glutamic acid in position 664 is exchanged for any other proteogenic amino acid except L-glutamic acid, and/or glycine in position 666 is exchanged for any other proteogenic amino acid except glycine.

The exchange in position 664 is preferably for L-lysine or L-arginine, especially L-lysine, and the exchange in position 666 is preferably for L-serine or L-threonine, especially L-serine.

SEQ ID No. 3 shows the base sequence of the pknD-1547 allele contained in the strain DM1547. The pknD-1547 allele codes for a protein whose amino acid sequence is shown in SEQ ID No. 4. The protein contains L-lysine in position 664 and L-serine in position 666. The DNA sequence of the pknD-1547 allele (SEQ ID No. 3) contains the base adenine in place of the base guanine contained in the pknD wild-type gene (SEQ ID No. 1) in position 2501, and the base adenine in place of the base guanine in position 2507.

Mutagenesis can be carried out by conventional methods using mutagenic substances such as N-methyl-N'-nitro-N-nitrosoguanidine or ultraviolet light. Mutagenesis can also be carried out using in vitro methods such as treatment with hydroxylamine (Miller, J.H.: A Short Course in Bacterial Genetics. A Laboratory Manual and Handbook for Escherichia coli and Related Bacteria, Cold Spring Harbor Laboratory Press, Cold Spring Harbor, 1992) or mutagenic oligonucleotides (T.A. Brown: Gentechnologie für Einsteiger (Gene Technology for Beginners), Spektrum Akademischer Verlag, Heidelberg, 1993), or the polymerase chain reaction (PCR) as described in the manual by Newton and Graham (PCR, Spektrum Akademischer Verlag, Heidelberg, 1994).

The corresponding alleles or mutations are sequenced and introduced into the chromosome by the method of gene replacement, for example as described in Peters-Wendisch et al. (Microbiology 144, 915-927 (1998)) for the pyc gene of C. glutamicum, in Schäfer et al. (Gene 145, 69-73 (1994)) for the hom-thrB gene region of C. glutamicum or in Schäfer et al. (Journal of Bacteriology 176, 7309-7319 (1994)) for the cg1 gene region of C. glutamicum. The corresponding alleles or the associated proteins can optionally be amplified in turn.

In addition it can be advantageous for the production of L-amino acids to overexpress not only the pknD gene but also one or more enzyme's of the particular biosynthetic pathway, the glycolysis, the anaplerosis, the citric acid cycle, the pentose phosphate cycle or the amino acid export, and optionally regulatory proteins.

Thus, for the production of L-amino acids, one or more endogenous genes selected from the following group can be overexpressed in addition to overexpression of the pknD gene:
- the dapA gene coding for dihydrodipicolinate synthase (EP-B-O 197 335),
- the gap gene coding for glyceraldehyde 3-phosphate dehydrogenase (Eikmanns (1992), Journal of Bacteriology 174, 6076-6086),
- the tpi gene coding for triose phosphate isomerase (Eikmanns (1992), Journal of Bacteriology 174, 6076-6086),
- the pgk gene coding for 3-phosphoglycerate kinase (Eikmanns (1992), Journal of Bacteriology 174, 6076-6086),
- the zwf gene coding for glucose-6-phosphate dehydrogenase (JP-A-09224661),
- the pyc gene coding for pyruvate carboxylase (DE-A-198 31 609),
- the lysC gene coding for a feedback-resistant aspartate kinase (Accession no. P26512; EP-B-0387527; EP-A-0699759),
- the lysE gene coding for lysine export (DE-A-195 48 222),
- The hom gene coding for homoserine dehydrogenase (EP-A-0131171),
- the ilvA gene coding for threonine dehydratase (Möckel et al., Journal of Bacteriology (1992) 8065-8072)) or the ilvA(fbr) allele coding for a feedback-resistant threonine dehydratase (Möckel et al., (1994), Molecular Microbiology 13, 833-842),
- the ilvBN gene coding for acetohydroxy acid synthase (EP-B-0356739),
- the ilvD gene coding for dihydroxy acid dehydratase (Sahm and Eggeling (1999), Applied and Environmental Microbiology 65, 1973-1979),
- the zwa1 gene coding for the Zwa1 protein (DE 199 59 328.0, DSM13115) .

In addition to overexpression of the pknD gene, it can also be advantageous for the production of L-amino acids to attenuate one or more genes selected from the following group:
- the pck gene coding for phosphoenol pyruvate carboxykinase (DE 199 50 409.1, DSM13047),
- the pgi gene coding for glucose-6-phosphate isomerase (US 09/396,478, DSM12969),
- the poxB gene coding for pyruvate oxidase (DE 199 51 975.7, DSM13114),
- the zwa2 gene coding for the Zwa2 protein (DE 199 59 327.2, DSM13113),
and, in particular, to reduce the expression.

In this context the term "attenuation" describes the reduction or switching-off of the intracellular activity, in a microorganism, of one or more enzymes (proteins) which are coded for by the appropriate DNA, for example by using a weak promoter or using a gene or allele coding for an appropriate enzyme with a low activity, or inactivating the appropriate gene or enzyme (protein), and optionally combining these measures.

By attenuation measures, the activity or concentration of the corresponding protein is in general reduced to 0 to 75%, 0 to 50%, 0 to 25%, 0 to 10% or 0 to 5% of the activity or concentration of the wild-type protein or of the activity or concentration of the protein in the starting microorganism.

It can also be advantageous for the production of amino acids not only to overexpress the pknD gene but also to switch off unwanted secondary reactions (Nakayama: "Breeding of Amino Acid Producing Micro-organisms", in: Overproduction of Microbial Products, Krumphanzl, Sikyta, Vanek (eds.), Academic Press, London, UK, 1982).

The microorganisms prepared according to the invention are also provided by the invention and can be cultivated for the production of amino acids continuously or discontinuously by the batch process, the fed batch process or the repeated fed batch process. A summary of known cultivation methods is described in the textbook by Chmiel (Bioprozesstechnik 1. Einführung in die Bioverfahrenstechnik (Bioprocess Technology 1. Introduction to Bioengineering) (Gustav Fischer Verlag, Stuttgart, 1991)) or in the textbook by Storhas (Bioreaktoren und periphere Einrichtungen (Bioreactors and Peripheral Equipment) (Vieweg Verlag, Brunswick/Wiesbaden, 1994)).

The culture medium to be used must appropriately meet the demands of the particular strains. Descriptions of culture media for various microorganisms can be found in "Manual of Methods for General Bacteriology" of the American Society for Bacteriology (Washington DC, USA, 1981).

Carbon sources which can be used are sugars and carbohydrates, e.g. glucose, sucrose, lactose, fructose, maltose, molasses, starch and cellulose, oils and fats, e.g. soybean oil, sunflower oil, groundnut oil and coconut fat, fatty acids, e.g. palmitic acid, stearic acid and linoleic acid, alcohols, e.g. glycerol and ethanol, and organic acids, e.g. acetic acid. These substances can be used individually or as a mixture.

Nitrogen sources which can be used are organic nitrogen-containing compounds such as peptones, yeast extract, meat extract, malt extract, corn steep liquor, soybean flour and urea, or inorganic compounds such as ammonium sulfate, ammonium chloride, ammonium phosphate, ammonium carbonate and ammonium nitrate. The nitrogen sources can be used individually or as a mixture.

Phosphorus sources which can be used are phosphoric acid, potassium dihydrogenphosphate or dipotassium hydrogenphosphate or the corresponding sodium salts. The culture medium must also contain metal salts, e.g. magnesium sulfate or iron sulfate, which are necessary for growth. Finally, essential growth-promoting substances such as amino acids and vitamins can be used in addition to the substances mentioned above. Suitable precursors can also be added to the culture medium. Said feed materials can be added to the culture all at once or fed in appropriately during cultivation.

The pH of the culture is controlled by the appropriate use of basic compounds such as sodium hydroxide, potassium hydroxide, ammonia or aqueous ammonia, or acidic compounds such as phosphoric acid or sulfuric acid. Foaming can be controlled using antifoams such as fatty acid polyglycol esters. The stability of plasmids can be maintained by adding suitable selectively acting substances, e.g. antibiotics, to the medium. Aerobic conditions are maintained by introducing oxygen or oxygen-containing gaseous mixtures, e.g. air, into the culture. The temperature of the culture is normally 20°C to 45°C and preferably 25°C to 40°C. The culture is continued until the formation of the desired product has reached a maximum. This objective is normally achieved within 10 hours to 160 hours.

Methods of determining L-amino acids are known from the state of the art. They can be analyzed for example by ion exchange chromatography with subsequent ninhydrin derivation, as described by Spackman et al. (Analytical Chemistry 30 (1958) 1190), or by reversed phase HPLC, as described by Lindroth et al. (Analytical Chemistry (1979) 51, 1167-1174).

A pure culture of the Corynebacterium glutamicum strain DM1547 was deposited as DSM 13994 in the Deutsche Sammlung für Mikroorganismen und Zellkulturen (German Collection of Microorganisms and Cell Cultures (DSMZ), Brunswick, Germany) on 16 January 2001 under the terms of the Budapest Treaty.

A pure culture of the Escherichia coli strain S17-1/pKl8mobsacB_pknD-XL was deposited as DSM 14410 in the Deutsche Sammlung für Mikroorganismen und Zellkulturen (German Collection of Microorganisms and Cell Cultures (DSMZ, Brunswick, Germany) on 18 July 2001 under the terms of the Budapest Treaty.

The fermentation process according to the invention is used for the preparation of amino acids.

The present invention is illustrated in greater detail below by means of Examples.

The isolation of plasmid DNA from Escherichia coli and all the techniques of restriction, Klenow treatment and alkaline phosphatase treatment were carried out according to Sambrook et al. (Molecular Cloning. A Laboratory Manual (1989), Cold Spring Harbor Laboratory Press, Cold Spring Harbor, NY, USA). Methods of transforming Escherichia coli are also described in this manual.

The composition of common nutrient media, such as LB or TY medium, can also be found in the manual by Sambrook et al.

### Example 1

### Preparation of a genomic cosmid gene library from Corynebacterium glutamicum ATCC13032

Chromosomal DNA from Corynebacterium glutamicum ATCC13032 was isolated as described by Tauch et al. (1995, Plasmid 33, 168-179) and partially cleaved with the restriction enzyme Sau3AI (Amersham Pharmacia, Freiburg, Germany, product description Sau3AI, code no. 27-0913-02). The DNA fragments were dephosphorylated with shrimp alkaline phosphatase (Roche Diagnostics GmbH, Mannheim, Germany, product description SAP, code no. 1758250). The DNA of cosmid vector SuperCos1 (Wahl et al. (1987), Proceedings of the National Academy of Sciences USA 84, 2160-2164), obtained from Stratagene (La Jolla, USA, product description SuperCosl Cosmid Vector Kit, code no. 251301), was cleaved with the restriction enzyme XbaI (Amersham Pharmacia, Freiburg, Germany, product description Xbal, code no. 27-0948-02) and also dephosphorylated with shrimp alkaline phosphatase.

The cosmid DNA was then cleaved with the restriction enzyme BamHI (Amersham Pharmacia, Freiburg, Germany, product description BamHI, code no. 27-0868-04). The cosmid DNA treated in this way was mixed with the treated ATCC13032 DNA and the mixture was treated with T4 DNA ligase (Amersham Pharmacia, Freiburg, Germany, product description T4 DNA ligase, code no. 27-0870-04). The ligation mixture was then packaged into phages using Gigapack II XL Packing Extract (Stratagene, La Jolla, USA, product description Gigapack II XL Packing Extract, code no. 200217).

For infection of the E. coli strain NM554 (Raleigh et al., 1988, Nucleic Acid Research 16, 1563-1575), the cells were taken up in 10 mM MgSO₄ and mixed with an aliquot of the phage suspension. Infection and titering of the cosmid library were carried out as described by Sambrook et al. (1989, Molecular Cloning: A Laboratory Manual, Cold Spring Harbor), the cells being plated on LB agar (Lennox, 1955, Virology 1, 190) containing 100 mg/l of ampicillin. After incubation overnight at 37°C, recombinant single clones were selected.

### Example 2

### Isolation and sequencing of the pknD gene

The cosmid DNA of a single colony was isolated with the Qiaprep Spin Miniprep Kit (product no. 27106, Qiagen, Hilden, Germany) in accordance with the manufacturer's instructions and partially cleaved with the restriction enzyme Sau3AI (Amersham Pharmacia, Freiburg, Germany, product description Sau3AI, product no. 27-0913-02). The DNA fragments were dephosphorylated with shrimp alkaline phosphatase (Roche Diagnostics GmbH, Mannheim, Germany, product description SAP, product no. 1758250). After separation by gel electrophoresis, the cosmid fragments in the size range from 1500 to 2000 bp were isolated with the QiaExII Gel Extraction Kit (product no. 20021, Qiagen, Hilden, Germany).

The DNA of sequencing vector pZero-1, obtained from Invitrogen (Groningen, The Netherlands, product description Zero Background Cloning Kit, product no. K2500-01), was cleaved with the restriction enzyme BamHI (Amersham Pharmacia, Freiburg, Germany, product description BamHI, product no. 27-0868-04). Ligation of the cosmid fragments into sequencing vector pZero-1 was carried out as described by Sambrook et al. (1989, Molecular Cloning: A Laboratory Manual, Cold Spring Harbor), the DNA mixture being incubated overnight with T4 ligase (Pharmacia Biotech, Freiburg, Germany). This ligation mixture was then introduced into the E. coli strain DHSαMCR (Grant, 1990, Proceedings of the National Academy of Sciences USA 87, 4645-4649) by electroporation (Tauch et al. 1994, FEMS Microbiol. Letters 123, 343-7) and plated on LB agar (Lennox, 1955, Virology 1, 190) containing 50 mg/l of zeocin.

Plasmid preparation of the recombinant clones was carried out with Biorobot 9600 (product no. 900200, Qiagen, Hilden, Germany). Sequencing was carried out by the dideoxy chain termination method of Sanger et al. (1977, Proceedings of the National Academy of Sciences USA 74, 5463-5467) with modifications by Zimmermann et al. (1990, Nucleic Acids Research 18, 1067). The "RR dRhodamin Terminator Cycle Sequencing Kit" from PE Applied Biosystems (product no. 403044, Weiterstadt; Germany) was used. Separation by gel electrophoresis and analysis of the sequencing reaction were carried out in a "Rotiphorese NF acrylamide/bisacrylamide" gel (29:1) (product no. A124.1, Roth, Karlsruhe, Germany) with the "ABI Prism 377" sequencer from PE Applied Biosystems (Weiterstadt, Germany).

The raw sequence data obtained were then processed using the Staden programming package (1986, Nucleic Acids Research 14, 217-231), version 97-0. The individual sequences of the pZero-1 derivatives were assembled into a cohesive contig. Computer-assisted coding region analysis was performed with the XNIP program (Staden, 1986, Nucleic Acids Research 14, 217-231).

The nucleotide sequence obtained is shown in SEQ ID No. 1. Analysis of the nucleotide sequence gave an open reading frame of 2223 base pairs, which was called the pknD gene. The pknD gene codes for a protein of 740 amino acids.

### Example 3

### Preparation of a replacement vector for replacement of the pknD allels

Chromosomal DNA was isolated from the strain DSM13994 by the method of Eikmanns et al. (Microbiology 140:1817-1828 (1994)). On the basis of the sequence of the pknD gene known for C. glutamicum from example 2, the following oligonucleotides were chosen for the polymerase chain reaction (see also SEQ ID No. 5 and SEQ ID No. 6):
pknD_XL-A1:
   5' (tct aga) cgg ttg gtg gtt cgg ttc ag 3'
pknD_XL-E1:
   5' (tct aga) agc ggc aat gcc ggt gag ta 3'

The primers shown were synthesized by MWG Biotech (Ebersberg, Germany) and the PCR reaction was carried out by the PCR method of Karreman (BioTechniques 24:736-742, 1998) with Pwo-Polymerase from Boehringer. The primers pknD_XL-A1 and pknD_XL-E1 each contain an inserted cleavage site for the restriction enzyme XbaI, these being indicated in parentheses in the representation. With the aid of the polymerase chain reaction, a 1.6 kb DNA section is amplified and isolated, this carrying the pknD gene or allele.

The amplified DNA fragment of approx. 1.6 kb length, which carries the pknD allele of the strain DSM13994, was cleaved with the restriction enzyme XbaI, identified by electrophoresis in a 0.8% agarose gel, isolated from the gel and purified by the conventional methods (QIAquick Gel Extraction Kit, Qiagen, Hilden).

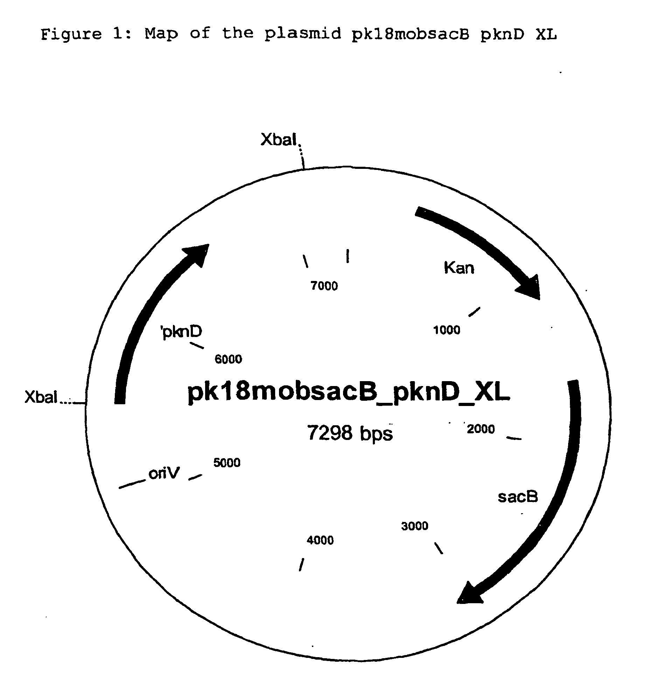

The plasmid pK18mobsacB (Jäger et al., Journal of Bacteriology, 1:784-791 (1992)) was also cleaved with the restriction enzyme XbaI. The plasmid pK18mobsacB and the PCR fragment were ligated. The E. coli strain S17-1 (Simon et al., 1993, Bio/Technology 1:784-791) was then electroporated with the ligation batch (Hanahan, In: DNA Cloning. A Practical Approach. Vol. I, IRL-Press, Oxford, Washington DC, USA, 1985). Selection of plasmid-carrying cells was carried out by plating out the transformation batch on LB Agar (Sambrook et al., Molecular cloning: a laboratory manual. 2^{nd} Ed. Cold Spring Harbor Laboratory Press, Cold Spring Harbor, N.Y., 1989), which had been supplemented with 25 mg/l kanamycin. Plasmid DNA was isolated from a transformant with the aid of the QIAprep Spin Miniprep Kit from Qiagen and checked by restriction with the restriction enzyme XbaI and subsequent agarose gel electrophoresis (0.8%). The plasmid was called pK18mobsacB_pknD_XL and is shown in Figure 1.

### Brief Description of the Figure:

Figure 1: Map of the plasmid pK18mobsacB_pknD_XL.

The abbreviations and designations used have the following meaning. The length data are to be understood as approx. values.
- sacB:: sacB gene
- oriV:: Replication origin V
- KmR:: Kanamycin resistance
- XbaI:: Cleavage site of the restriction enzyme XbaI
- pknD':: Incomplete fragment of the pknD gene from DM1547

## Claims

1. Coryneform bacteria, transformed with a polynucleotide from coryneform bacterium wherein said polynucleotide encodes a polypeptide having an amino acid sequence which is at least 90 % identical to the amino acid sequence set out in SEQ ID NO:2 or having an amino acid sequence of the variant of SEQ ID NO:2 with amino acid exchanges in the section between position 661 and 669, and wherein said polynucleotide is overexpressed.

2. Bacteria according to claim 1, wherein said amino acid exchanges are selected from the group:
a) SEQ ID NO:2 wherein the glutamic acid at position 664 is exchanged; or
b) SEQ ID NO: 2 wherein the glycine at position 666 is exchanged; or
c) SEQ ID NO:2 wherein the glutamic acid at position 664 and glycine at position 666 are exchanged.

3. Bacteria according to claim 2, wherein the glutamic acid at position 664 of SEQ ID NO: 2 is replaced by L-lysine or L-arginine.

4. Bacteria according to claim 2, wherein the glycine at position 666 of SEQ ID NO:2 is replaced by L-serine or L-threonine.

5. Bacteria according to claim 2, wherein the glutamic acid at position 664 is replaced by L-lysine and the glycine at position 666 of SEQ ID NO:2 is replaced by L-serine.

6. Bacteria according to claim 1 wherein the polynucleotide is chosen from the group:
a) nucleotide sequences as set out in SEQ ID NO: 1 nucleotides 512 to 2731 or SEQ ID NO:3 nucleotides 512 2 to 2731; or
b) nucleotide sequences as set out in SEQ. ID NO:1 or SEQ ID NO: 3 ; or
c) nucleotide sequence corresponding to nucleotides 512 to 2731 of sequence SEQ ID NO:1 or nucleotides 512 to 2731 of sequence SEQ ID NO:3 within the degeneracy of the genetic code; or
d) nucleotide sequences with neutral sense mutations in SEQ ID NO:1 nucleotides 512 to 2731 or SEQ ID NO: 3 nucleotides 512 to 2731.

7. Bacteria according to claim 1, wherein overexpression is achieved by a method chosen from the group:
a) increasing the copy number of said polynucleotide, or
b) using a promoter; or
c) introducing said polynucleotide into the chromosome.

8. A process for the preparation of L-amino acids, comprising
a) fermenting of the bacteria according to one or more of the claims 1 to 7,
b) enrichment of the L-amino acid in the medium or in the cells of the bacteria.

9. The process according to claim 8, wherein coryneform bacteria are fermented wherein one or more enzymes has (have) increased activity or concentration by at least 10% based on that of the protein of the starting microorganism, whereby these enzymes are encoded by endogenous genes, chosen from the group:
a) the dapA gene coding for dihydrodipicolinate synthase,
b) the gap gene coding for glyceraldehyde 3-phosphate dehydrogenase,
c) the tpi gene coding for triose phosphate isomerase,
d) the pgk gene coding for 3-phosphoglycerate kinase,
e) the zwf gene coding for glucose-6-phosphate dehydrogenase,
f) the pyc gene coding for pyruvate carboxylase,
g) the lysc gene coding for a feedback-resistant aspartate kinase,
h) the lysE gene coding for a protein for lysine export,
i) the hom gene coding for homoserine dehydrogenase,
j) the ilvA gene coding for threonine dehydratase or the ilvA(Fbr) allele coding for afeedback-resistantthreonine dehydratase,
k) the ilvBN gene coding for acetohydroxy acid synthase,
l) the ilvD gene coding for dihydroxy acid dehydratase, or
m) the zwa1 gene coding for the Zwa1 protein.

10. The process according to claims 8 or 9, wherein coryneform bacteria are fermented in which one or more genes selected from the following group are simultaneously attenuated and the activity orconcentration of the corresponding protein is reduced to 0 to 75 % of the activity or concentration based on that of the protein in the starting microorganisms:
a) the pck gene coding for phosphoeriol pyruvate carboxykinase,
b) the pgi gene coding for glucose-6-phosphate isomerase,
c): the poxB gene coding for pyruvate oxidase, or
d) the zwa2 gene coding for the Zwa2 protein.

11. The process according to one or more of claims 8 - 10, wherein microorganisms of the species Corynebacterium glutamicum are used.

12. The process according to claim 8, wherein a Corynebacterium strain is used which is transformed with the vector obtainable from DSM14410, deposited at the Deutsche Sammlung für Mikroorganismen und Zelikulturen (German Collection of Microorganisms and Cell Cultures), Brunswick, Germany.

13. The process according to one or more of the claims 8 to 12, wherein L-lysine is produced.

14. A polynucleotide originating from corynebacteria and encoding protein kinase D of SEQ ID NO:2, wherein the corresponding amino acid sequences between positions 661 and 669 of SEQ ID NO:2 are modified by amino acid exchange selected from the group:
a) any other proteogenic amino-acid except glutamic acid in position 664; or
b) L-lysine or L-arginine in positions 664; or
c) any other proteogenic amino acid except glycine in position 666; or
d) L-serine or L-threonine In position 666; or
e) L-lysine in position 664 and L-serine in positions 666.

15. The polynucleotide according to claim 14, wherein said DNA contains the nudeobase adenine in position 2501 and the nucleobase adenine in position 2507 of SEQ ID NO:1 as shown in SEQ ID NO:3.

16. Escherichia coli strain S17-1/pk18mobsacB_pknD_XL as DSM 14410 deposited at the Deutsche Sammlung fur Mikroorganismen und Zeilkulturen (German Collection of Microorganisms and Cell Cultures), Brunswick, Germany.

17. A method of detecting RNA, cDNA and.DNA in order to isolate nucleic acids, or polynucleotides or genes, which code for protein kinase D with SEQ ID NO:4, wherein the polynucleotide containing the polynucleotide sequence SEQ ID NO: 3 is used as hybridization probe.

18. The method according to claim 17 wherein arrays, micro-arrays or DNA chips are used.

## Patentansprüche

1. Coryneforme Bakterien, die mit einem Polynucleotid aus einem coryneformen Bakterium transformiert sind, wobei das Polynucleotid ein Polypeptid mit einer Aminosäuresequenz codiert, die zu mindestens 90% identisch zu der in SEQ ID NR: 2 dargestellten Aminosäuresequenz ist oder eine Aminosäuresequenz der Variante von SEQ ID NR: 2 mit Aminosäureaustauschen im Abschnitt zwischen Position 661 und 669 besitzt, und wobei das Polynucleotid überexprimiert wird.

2. Bakterien nach Anspruch 1, wobei die Aminosäureaustausche aus der folgenden Gruppe ausgewählt sind:
a) SEQ ID NR: 2, wobei die Glutaminsäure an Position 664 ausgetauscht ist, oder
b) SEQ ID NR: 2, wobei das Glycin an Position 666 ausgetauscht ist, oder
c) SEQ ID NR: 2, wobei die Glutaminsäure an Position 664 und das Glycin an Position 666 ausgetauscht sind.

3. Bakterien nach Anspruch 2, wobei die Glutaminsäure an Position 664 der SEQ ID NR: 2 durch L-Lysin oder L-Arginin ausgetauscht ist.

4. Bakterien nach Anspruch 2, wobei das Glycin an Position 666 der SEQ ID NR: 2 durch L-Serin oder L-Threonin ausgetauscht ist.

5. Bakterien nach Anspruch 2, wobei die Glutaminsäure an Position 664 durch L-Lysin und das Glycin an Position 666 der SEQ ID NR: 2 durch L-Serin ausgetauscht sind.

6. Bakterien nach Anspruch 1, wobei das Polynucleotid aus der folgenden Gruppe ausgewählt ist:
a) Nucleotidsequenzen, wie in SEQ ID NR: 1 Nucleotide 512 bis 2731 oder SEQ ID NR: 3 Nucleotide 512 bis 2731 dargestellt, oder
b) Nucleotidsequenzen, wie in SEQ ID NR: 1 oder SEQ ID NR: 3 dargestellt, oder
c) Nucleotidsequenzen, die den Nucleotiden 512 bis 2731 der Sequenz SEQ ID NR: 1 oder den Nucleotiden 512 bis 2731 der Sequenz SEQ ID NR: 3 innerhalb der Degeneration des genetischen Codes entsprechen, oder
d) Nucleotidsequenzen mit neutralen Sense-Mutationen in SEQ ID NR: 1 Nucleotide 512 bis 2731 oder SEQ ID NR: 3 Nucleotide 512 bis 2731.

7. Bakterien nach Anspruch 1, wobei eine Überexpression durch ein Verfahren erzielt wird, das aus der folgenden Gruppe ausgewählt ist:
a) Erhöhen der Kopienzahl des Polynucleotids oder
b) Verwenden eines Promotors oder
c) Einfügen des Polynucleotids in das Chromosom.

8. Verfahren zur Herstellung von L-Aminosäuren, das Folgendes umfasst:
a) Fermentieren der Bakterien nach einem oder mehreren der Ansprüche 1 bis 7,
b) Anreichern der L-Aminosäure im Medium oder in den Zellen der Bakterien.

9. Verfahren nach Anspruch 8, wobei coryneforme Bakterien fermentiert werden, wobei ein oder mehrere Enzyme eine um mindestens 10% erhöhte Aktivität oder Konzentration, bezogen auf diejenige des Proteins des Ausgangsmikroorganismus, besitzen, wobei diese Enzyme von endogenen Genen codiert werden, die aus der folgenden Gruppe ausgewählt sind:
a) dem dapA-Gen, das Dihydrodipicolinatsynthase codiert,
b) dem gap-Gen, das Glycerinaldehyd-3-phosphat-dehydrogenase codiert,
c) dem tpi-Gen, das Triosephosphatisomerase codiert,
d) dem pgk-Gen, das 3-Phosphoglyceratkinase codiert,
e) dem zwf-Gen, das Glucose-6-phosphatdehydrogenase codiert,
f) dem pyc-Gen, das Pyruvatcarboxylase codiert,
g) dem lysC-Gen, das eine rückkopplungsresistente Aspartatkinase codiert,
h) dem lysE-Gen, das ein Protein für den Lysin-Export codiert,
i) dem hom-Gen, das Homoserindehydrogenase codiert,
j) dem ilvA-Gen, das Threonindehydratase codiert, oder dem ilvA(Fbr)-Allel, das eine rückkopplungsresistente Threonindehydratase codiert,
k) dem ilvBN-Gen, das Acetohydroxysäuresynthase codiert,
l) dem ilvD-Gen, das Dihydroxysäuredehydratase codiert, oder
m) dem zwal-Gen, das das Zwal-Protein codiert.

10. Verfahren nach den Ansprüchen 8 oder 9, wobei coryneforme Bakterien fermentiert werden, in denen ein oder mehrere Gene, ausgewählt aus der folgenden Gruppe, gleichzeitig abgeschwächt sind und die Aktivität oder Konzentration des entsprechenden Proteins auf 0 bis 75% der Aktivität oder Konzentration, bezogen auf diejenige des Proteins in den Ausgangsmikroorganismen, verringert ist:
a) das pck-Gen, das Phosphoenolpyruvatcarboxykinase codiert,
b) das pgi-Gen, das Glucose-6-phosphatisomerase codiert,
c) das poxB-Gen, das Pyruvatoxidase codiert, oder d) das zwa2-Gen, das das Zwa2-Protein codiert.

11. Verfahren nach einem oder mehreren der Ansprüche 8 bis 10, wobei Mikroorganismen der Spezies Corynebacterium glutamicum verwendet werden.

12. Verfahren nach Anspruch 8, wobei ein Corynebacterium-Stamm verwendet wird, der mit dem Vektor transformiert ist, der erhältlich ist aus DSM14410, hinterlegt bei der Deutschen Sammlung für Mikroorganismen und Zellkulturen, Braunschweig, Deutschland.

13. Verfahren nach einem oder mehreren der Ansprüche 8 bis 12, wobei L-Lysin produziert wird.

14. Polynucleotid, das aus Corynebakterien stammt und die Proteinkinase D der SEQ ID NR: 2 codiert, wobei die entsprechenden Aminosäuresequenzen zwischen den Positionen 661 und 669 der SEQ ID NR: 2 modifiziert sind durch Aminosäureaustausch, ausgewählt aus der folgenden Gruppe:
a) jede andere proteogene Aminosäure, ausgenommen Glutaminsäure, an Position 664 oder
b) L-Lysin oder L-Arginin an Position 664 oder
c) jede andere proteogene Aminosäure, ausgenommen Glycin, an Position 666 oder
d) L-Serin oder L-Threonin an Position 666 oder
e) L-Lysin an Position 664 und L-Serin an Position 666.

15. Polynucleotid nach Anspruch 14, wobei die DNA die Nucleobase Adenin an Position 2501 und die Nucleobase Adenin an Position 2507 der SEQ ID NR: 1 enthält, wie in SEQ ID NR: 3 gezeigt.

16. Escherichia coli-Stamm S17-1/pK18mobsacB-pknD-XL, hinterlegt als DSM14410 bei der Deutschen Sammlung für Mikroorganismen und Zellkulturen, Braunschweig, Deutschland.

17. Verfahren zum Nachweis von RNA, cDNA und DNA, um Nucleinsäuren oder Polynucleotide oder Gene zu isolieren, die Proteinkinase D mit der SEQ ID NR: 4 codieren, wobei das Polynucleotid, das die Polynucleotidsequenz SEQ ID NR: 3 enthält, als Hybridisierungssonde verwendet wird.

18. Verfahren nach Anspruch 17, wobei Arrays, Mikroarrays oder DNA-Chips verwendet werden.

## Revendications

1. Bactéries coryneformes, transformées par un polynucléotide d'une bactérie coryneforme, dans lesquelles ledit polynucléotide code pour un polypeptide ayant une séquence d'acides aminés qui a au moins 90% d'identité avec la séquence d'acides aminés représentée dans la SEQ ID NO:2 ou ayant une séquence d'acides aminés du variant de la SEQ ID NO:2 avec des échanges d'acides aminés dans la section entre les positions 661 et 669, et dans lesquelles ledit polynucléotide est surexprimé.

2. Bactéries selon la revendication 1, dans lesquelles lesdits échanges d'acides aminés sont sélectionnés dans le groupe suivant :
a) SEQ ID NO:2 dans laquelle l'acide glutamique en position 664 est échangé; ou
b) SEQ ID NO:2 dans laquelle la glycine en position 666 est échangée; ou
c) SEQ ID NO:2 dans laquelle l'acide glutamique en position 664 et la glycine en position 666 sont échangés.

3. Bactéries selon la revendication 2, dans lesquelles l'acide glutamique en position 664 de la SEQ ID NO:2 est remplacé par la L-lysine ou la L-arginine.

4. Bactéries selon la revendication 2, dans lesquelles la glycine en position 666 de la SEQ ID NO:2 est remplacée par la L-sérine ou la L-thréonine.

5. Bactéries selon la revendication 2, dans lesquelles l'acide glutamique en position 664 est remplacé par la L-lysine et la glycine en position 666 de la SEQ ID NO:2 est remplacée par la L-sérine.

6. Bactéries selon la revendication 1, dans lesquelles le polynucléotide est choisi dans le groupe suivant :
a) séquences nucléotidiques représentées dans la SEQ ID NO:1, nucléotides 512 à 2731, ou la SEQ ID NO:3, nucléotides 512 à 2731; ou
b) séquences nucléotidiques représentées dans la SEQ ID NO:1 ou la SEQ ID NO:3; ou
c) séquences nucléotidiques correspondant aux nucléotides 512 à 2731 de la SEQ ID NO:1 ou aux nucléotides 512 à 2731 de la SEQ ID NO:3, dans les limites de la dégénérescence du code génétique; ou
d) séquences nucléotidiques avec des mutations à sens neutre dans la SEQ ID NO:1, nucléotides 512 à 2731, ou la SEQ ID NO:3, nucléotides 512 à 2731.

7. Bactéries selon la revendication 1, dans lesquelles la surexpression est obtenue par un procédé choisi dans le groupe suivant :
a) augmentation du nombre de copies dudit polynucléotide; ou
b) utilisation d'un promoteur; ou
c) introduction dudit polynucléotide dans le chromosome.

8. Procédé pour la préparation d'acides L-aminés, comprenant
a) la fermentation des bactéries selon une ou plusieurs des revendications 1 à 7,
b) l'enrichissement des acides L-aminés dans le milieu ou dans les cellules bactériennes.

9. Procédé selon la revendication 8, dans lequel les bactéries coryneformes ont fermenté, fermentation dans laquelle une ou plusieurs enzymes a (ont) une activité ou une concentration accrue d'au moins 10% par rapport à celle de la protéine du micro-organisme de départ, ce par quoi ces enzymes sont codées par des gènes endogènes, choisis dans le groupe suivant :
a) le gène dapA codant pour la dihydropicolinate synthétase,
b) le gène gap codant pour la glycéraldéhyde-3-phosphate déshydrogénase,
c) le gène tpi codant pour la triose phosphate isomérase,
d) le gène pgk codant pour la 3-phosphoglycérate kinase,
e) le gène zwf codant pour la glucose-6-phosphate déshydrogénase,
f) le gène pyc codant pour la pyruvate carboxylase,
g) le gène lysC codant pour une aspartate kinase résistante à la rétroaction,
h) le gène lysE codant pour une protéine pour l'exportation de la lysine,
i) le gène hom codant pour l'homosérine déshydrogénase,
j) le gène ilvA codant pour la théronine déshydratase ou l'allèle ilvA(Fbr) codant pour une thréonine déshydratase résistante à la rétroaction,
k) le gène ilvBN codant pour l'acétohydroxyacide synthétase,
l) le gène ilvD codant pour la dihydroxyacide déshydratase,
m) le gène zwa1 codant pour la protéine zwa1.

10. Procédé selon les revendications 8 ou 9, dans lequel les bactéries coryneformes ont fermenté, fermentation dans laquelle un ou plusieurs gènes sélectionnés dans le groupe suivant sont simultanément atténués et l'activité ou la concentration de la protéine correspondante est réduite à 0 à 75% de l'activité ou de la concentration par rapport à celle de la protéine dans les micro-organismes de départ :
a) le gène pck codant pour la phosphoénolpyruvate carboxykinase,
b) le gène pgi codant pour la glucose-6-phosphate isomérase,
c) le gène poxB codant pour la pyruvate oxydase, ou
d) le gène zwa2 codant pour la protéine zwa2.

11. Procédé selon une ou plusieurs des revendications 8 à 10, dans lequel des micro-organismes de l'espèce Corynebacterium glutamicum sont utilisés.

12. Procédé selon la revendication 8, dans lequel est utilisée une souche de Corynebacterium qui est transformée avec le vecteur pouvant être obtenu de DSM14410, déposé auprès de la Deutsche Sammlung für Mikroorganismen und Zellkulturen (Collection allemande de micro-organismes et de cultures cellulaires), Brunswick, Allemagne.

13. Procédé selon l'une quelconque des revendications 8 à 12, dans lequel de la L-lysine est produite.

14. Polynucléotide issu de corynebactéries et codant pour la protéine kinase D de la SEQ ID NO:2, dans lequel les séquences d'acides aminés correspondant aux positions 661 à 669 de la SEQ ID NO:2 sont modifiées par des échanges d'acides aminés sélectionnés dans le groupe suivant :
a) tout autre acide aminé protéogène à l'exception de l'acide glutamique en position 664; ou
b) la L-lysine ou la L-arginine en position 664; ou
c) tout autre acide aminé protéogène à l'exception de la glycine en position 666; ou
d) la L-sérine ou la L-thréonine en position 666; ou
e) la L-lysine en position 664 et la L-sérine en position 666.

15. Polynucléotide selon la revendication 14, dans lequel ledit ADN contient la nucléobase adénine en position 2501 et la nucléobase adénine en position 2507 de la SEQ ID NO:1, comme le montre la SEQ ID NO:3.

16. Escherichia coli souche S17-1/pK18mobsacB_pknD_XL, déposée sous la référence DSM 14410 auprès de la Deutsche Sammlung für Mikroorganismen und Zellkulturen (Collection allemande de micro-organismes et de cultures cellulaires), Brunswick, Allemagne.

17. Procédé pour détecter l'ARN, l'ADNc et l'ADN afin d'isoler des acides nucléiques ou des polynucléotides ou des gènes qui codent pour la protéine kinase D avec la SEQ ID NO:4, dans lequel le polynucléotide contenant la séquence polynucléotidique de SEQ ID NO:3 est utilisé comme sonde d'hybridation.

18. Procédé selon la revendication 17, dans lequel sont utilisés des formations, des microformations ou des puces d'ADN.


### SEQUENCE LISTING

<110> Degussa AG
<120> Nucleotide sequences coding for the pknD gene
<130> 000507 BT
<140>
   <141>
<160> 6
<170> PatentIn Ver. 2.1
<210> 1
   <211> 3341
   <212> DNA
   <213> Corynebacterium glutamicum
<220>
   <221> CDS
   <222> (512) .. (2731)
   <223> pknD gene
<400> 1
   agaacgccat tgcttgagcg cgtcgcataa cttcacgagc caactggcca tgaagtgcat 60
   cgatggggcg accaggaagg gtctcgtctt cggtaaacag gaacgcgagg atttcctcgt 120
   cgctgaagcc accgtcggca agcaaggcaa taactccagg gatgaaacgg ttggtgtttt 180
   ccttcttggt gctcaggaaa gcttctggaa tgtagcgaat accgtcgcgc cggaccacga 240
   tcaatttgtg ttcattgacc agatccatca ccttggtgac aacaacgccg aggcgctcgg 300
   ctgtctccgg aagggtcagc aatggttcat tgtcgggcag ggcgaaggaa gattcattgt 360
   tggaactcac agtcttaatt tagctggttc gagctctaat ggagaatctt tagggtattt 420
   ctgcgcgtgc cgggaatgaa agcaccttct tgacctttga aaacaggatg tcactaccac 480
   tttttgtgta ccttccgaca tactggaacg c atg gca aac ttg aag gtc ggt    532
                                      Met Ala Asn Leu Lys Val Gly
                                      1               5
   gac gtt tta gag gac agg tat cgg att gaa act ccg att gcc cgg ggt   580
   Asp Val Leu Glu Asp Arg Tyr Arg Ile Glu Thr Pro Ile Ala Arg Gly
           10                  15                  20
   ggt atg tct acc gtg tac agg tgc ctt gat ctt cgt tta gga cgt tcc   628
   Gly Met Ser Thr Val Tyr Arg Cys Leu Asp Leu Arg Leu Gly Arg Ser
       25                  30                  35
   atg gcg ctt aaa gtc atg gaa gaa gat ttc gtt gat gat ccc att ttc   676
   Met Ala Leu Lys Val Met Glu Glu Asp Phe Val Asp Asp Pro Ile Phe
   40                  45                  50                  55
   cgg cag cgt ttc cgt agg gaa gct cgg tca atg gcg cag cta aat cat     724
   Arg Gln Arg Phe Arg Arg Glu Ala Arg Ser Met Ala Gln Leu Asn His
                   60                  65                  70
   cca aat ttg gtc aat gtg tat gat ttt tcc gct act gac ggt ttg gtg     772
   Pro Asn Leu Val Asn Val Tyr Asp Phe Ser Ala Thr Asp Gly Leu Val
               75                  80                  85
   tat ctg gtg atg gag tta atc act ggt ggc acc ttg cgt gag ttg ctg     820
   Tyr Leu Val Met Glu Leu Ile Thr Gly Gly Thr Leu Arg Glu Leu Leu
           90                  95                  100
   gct gag cgg gga cct atg ccc ccg cat gct gct gtg ggc gtt atg cgt     868
   Ala Glu Arg Gly Pro Met Pro Pro His Ala Ala Val Gly Val Met Arg
       105                 110                 115
   ggg gtg ctc acg ggt ctc gcg gct gcc cac cgg gcg ggc atg gtg cac     916
   Gly Val Leu Thr Gly Leu Ala Ala Ala His Arg Ala Gly Met Val His
   120                 125                 130                 135
   cgg gat atc aag cct gac aac gtg ttg atc aat agt gat cac cag gtg     964
   Arg Asp Ile Lys Pro Asp Asn Val Leu Ile Asn Ser Asp His Gln Val
                   140                 145                 150
   aaa ctg tct gat ttc ggc ttg gtt cga gcg gct cac gcc ggc cag tct    1012
   Lys Leu Ser Asp Phe Gly Leu Val Arg Ala Ala His Ala Gly Gln Ser
               155                 160                 165
   cag gac aat cag att gtg ggc acg gtg gct tat ctt tcc cct gag cag    1060
   Gln Asp Asn Gln Ile Val Gly Thr Val Ala Tyr Leu Ser Pro Glu Gln
           170                 175                 180
   gtt gag ggc ggt gag atc ggg ccg gcc agc gac gtg tat tcg gca ggc    1108
   Val Glu Gly Gly Glu Ile Gly Pro Ala Ser Asp Val Tyr Ser Ala Gly
       185                 190                 195
   att gtg ctc ttt gag ctg ctc aca ggc acc acg cct ttt tcg ggc gag    1156
   Ile Val Leu Phe Glu Leu Leu Thr Gly Thr Thr Pro Phe Ser Gly Glu
   200                 205                 210                 215
   gat gat ctc gac cat gca tac gcc cgc ctt acg gaa gtc gtg ccg gca    1204
   Asp Asp Leu Asp His Ala Tyr Ala Arg Leu Thr Glu Val Val Pro Ala
                   220                 225                 230
   ccg agt tcg ctt atc gac ggc gtc ccc tcc ctc atc gat gag ctt gtc    1252
   Pro Ser Ser Leu Ile Asp Gly Val Pro Ser Leu Ile Asp Glu Leu Val
               235                 240                 245
   gcg aca gct acc tcc att aat cct gag gat cgt ttc gat gat tct gga    1300
   Ala Thr Ala Thr Ser Ile Asn Pro Glu Asp Arg Phe Asp Asp Ser Gly
           250                 255                 260
   gag ttt ttg tcc gca ctg gaa gat gtc gca aca gag ttg agc ttg ccg    1348
   Glu Phe Leu Ser Ala Leu Glu Asp Val Ala Thr Glu Leu Ser Leu Pro
       265                 270                 275
   gct ttc cgg gtc cct gtg ccg gtt aat tcc gca gcc aat agg gct aat    1396
   Ala Phe Arg Val Pro Val Pro Val Asn Ser Ala Ala Asn Arg Ala Asn
   280                 285                 290                 295
   gcc cag gtc ccg gat gct cag cca act gat atg ttt acc acc cat atc    1444
   Ala Gln Val Pro Asp Ala Gln Pro Thr Asp Met Phe Thr Thr His Ile
                   300                 305                 310
   ccc aag act cct gag cct gat cac act gcg atc att ccg gtg gcc tca    1492
   Pro Lys Thr Pro Glu Pro Asp His Thr Ala Ile Ile Pro Val Ala Ser
               315                 320                 325
   gca aat gag acg tcg att ctg cct gcg caa aac atg gca caa aat atg    1540
   Ala Asn Glu Thr Ser Ile Leu Pro Ala Gln Asn Met Ala Gln Asn Met
           330                 335                 340
   gcg cag aat ccg ctg caa cct ccg gaa cct gat ttc gcc ccg gag cca    1588
   Ala Gln Asn Pro Leu Gln Pro Pro Glu Pro Asp Phe Ala Pro Glu Pro
       345                 350                 355
   cct ccg gac aca gcg ctg aat att caa gat caa gag ctt gcg cgc gcc    1636
   Pro Pro Asp Thr Ala Leu Asn Ile Gln Asp Gln Glu Leu Ala Arg Ala
   360                 365                 370                 375
   gat gag cca gaa att aat acc gtc agc aat cgt tcc aaa ttg aag ctg    1684
   Asp Glu Pro Glu Ile Asn Thr Val Ser Asn Arg Ser Lys Leu Lys Leu
                   380                 385                 390
   acg ttg tgg tca att ttc gtg gtc gca gtg atc gct gct gtt gct gtt    1732
   Thr Leu Trp Ser Ile Phe Val Val Ala Val Ile Ala Ala Val Ala Val
               395                 400                 405
   ggc ggt tgg tgg ttc ggt tca ggc cgt tac ggt gag att ccg cag gtg    1780
   Gly Gly Trp Trp Phe Gly Ser Gly Arg Tyr Gly Glu Ile Pro Gln Val
           410                 415                 420
   ttg ggc atg gat gag gtc cag gca gta gct gtt gta gag gaa gct ggt    1828
   Leu Gly Met Asp Glu Val Gln Ala Val Ala Val Val Glu Glu Ala Gly
       425                 430                 435
   ttc gtg gca gtg gct gaa cct cag tat gac aat gag gtt ccc act ggt    1876
   Phe Val Ala Val Ala Glu Pro Gln Tyr Asp Asn Glu Val Pro Thr Gly
   440                 445                 450                 455
   tcg att att ggg act gaa cct tct ttt ggt gag cgc ctt cct cgc ggc    1924
   Ser Ile Ile Gly Thr Glu Pro Ser Phe Gly Glu Arg Leu Pro Arg Gly
                   460                 465                 470
   gag gat gtt tct gtc ctc gtc tct caa ggg cgt ccc gtg gtg ccg gat    1972
   Glu Asp Val Ser Val Leu Val Ser Gln Gly Arg Pro Val Val Pro Asp
               475                 480                 485
   ctt agc gag gat cga tcc tta agc acc gtt cgt gaa gag ttg gaa cag    2020
   Leu Ser Glu Asp Arg Ser Leu Ser Thr Val Arg Glu Glu Leu Glu Gln
           490                 495                 500
   cgc acg ttc gtc tgg gtt gat ggc cca ggt gaa tat tct gac gat gtt    2068
   Arg Thr Phe Val Trp Val Asp Gly Pro Gly Glu Tyr Ser Asp Asp Val
       505                 510                 515
   cca gaa gga caa gta gtt tct ttt aca ccg tcg tca ggc acg cag ctt    2116
   Pro Glu Gly Gln Val Val Ser Phe Thr Pro Ser Ser Gly Thr Gln Leu
   520                 525                 530                 535
   gat gtt ggt gaa acc gtg cag atc cat ttg agc cga ggc ccc gcc ccg    2164
   Asp Val Gly Glu Thr Val Gln Ile His Leu Ser Arg Gly Pro Ala Pro
                   540                 545                 550
   gtt gag att cct gat gtc tct ggc atg gga gtg gat cag gca aca cgt    2212
   Val Glu Ile Pro Asp Val Ser Gly Met Gly Val Asp Gln Ala Thr Arg
               555                 560                 565
   gtg ttg gag cgc gca ggt ttg agc gtc gag cgt act gaa gaa ggc ttt    2260
   Val Leu Glu Arg Ala Gly Leu Ser Val Glu Arg Thr Glu Glu Gly Phe
           570                 575                 580
   gat gct gag aca cca aat ggt gat gtc tac ggg act tcg ccc aag gta    2308
   Asp Ala Glu Thr Pro Asn Gly Asp Val Tyr Gly Thr Ser Pro Lys Val
       585                 590                 595
   tct act gag gtc aag cgc gga acc tct gtt gtg ctg cag gtg tcc aat    2356
   Ser Thr Glu Val Lys Arg Gly Thr Ser Val Val Leu Gln Val Ser Asn
   600                 605                 610                 615
   gct att tcg gta ccg gat gtg gtg ggt atg acc aag gac gaa gcc acc    2404
   Ala Ile Ser Val Pro Asp Val Val Gly Met Thr Lys Asp Glu Ala Thr
                   620                 625                 630
   gcg gcg ctt gcg gaa gaa gga ttg gtc gtg gcg tcg aca agc att att    2452
   Ala Ala Leu Ala Glu Glu Gly Leu Val Val Ala Ser Thr Ser Ile Ile
               635                 640                 645
   cct ggt gag gcg gcg agc tcc gct gac gcc gtc gtg acc gtc gag cct    2500
   Pro Gly Glu Ala Ala Ser Ser Ala Asp Ala Val Val Thr Val Glu Pro
           650                 655                 660
   gaa tcc ggc agc cgc gtt gat cca gcg cat ccg cag gtc agc ctc ggg    2548
   Glu Ser Gly Ser Arg Val Asp Pro Ala His Pro Gln Val Ser Leu Gly
       665                 670                 675
   tta gct ggg gag att caa gtt cca agc gtg gtt gga cgt aag gtt agc    2596
   Leu Ala Gly Glu Ile Gln Val Pro Ser Val Val Gly Arg Lys Val Ser
   680                 685                 690                 695
   gat gct cga agc att ctg gaa gaa gcc ggt tta acg ctg aca act gat    2644
   Asp Ala Arg Ser Ile Leu Glu Glu Ala Gly Leu Thr Leu Thr Thr Asp
                   700                 705                 710
   gcg gac gac aac gat cga att tat agt caa acc cct cgt gca cgc agc    2692
   Ala Asp Asp Asn Asp Arg Ile Tyr Ser Gln Thr Pro Arg Ala Arg Ser
               715                 720                 725
   gaa gtc tcg gta ggg gga gaa gtt aca gta agg gcg ttt tagtggttcc     2741
   Glu Val Ser Val Gly Gly Glu Val Thr Val Arg Ala Phe
           730                 735                 740
   ctcgttgcag caatggcgaa aacctgctct catcctggcc attttgacgg tgctaggcgt 2801
   actcctgacc cattggttcg cctggccact cacctggccg ctggggctgc gtcttcccgt 2861
   tgatgtagag gtgtactggc agggtgcgcg cgagttttgg ctcgccgatg atctctacga 2921
   catcaggtat gacaccactt tcgacaactt gccgttcacc tatcccccct tcggtgcgtt 2981
   ggtgttcacc ccattgtggt ggattcatga cctctttggt cttctcgtca ccgaacgtgt 3041
   cttcgcgcta atcacgctgc tcaccaccta cgctgtggca gttttcctgc tccgcctggc 3101
   cggcgtgcgc gatcgtgtgt gggaattcgt cgcattcgca gccctgctcg tgtccgcgcc 3161
   ggtgtatttc acactcaata ttgggcaaat aaacgtcatg ctcatggctt taacgctttt 3221
   cgacgtcgcc ctcccccgca gcacgcgcca ttcaggcgtg ctcaaatacg tgccactcgg 3281
   cgtactcacc ggcattgccg ctgcgatcaa actaacccca ctagtgttcg ggctgtattt 3341
<210> 2
   <211> 740
   <212> PRT
   <213> Corynebacterium glutamicum
<400> 2
   Met Ala Asn Leu Lys Val Gly Asp Val Leu Glu Asp Arg Tyr Arg Ile
   1               5                   10                  15
   Glu Thr Pro Ile Ala Arg Gly Gly Met Ser Thr Val Tyr Arg Cys Leu
               20                  25                  30
   Asp Leu Arg Leu Gly Arg Ser Met Ala Leu Lys Val Met Glu Glu Asp
           35                  40                  45
   Phe Val Asp Asp Pro Ile Phe Arg Gln Arg Phe Arg Arg Glu Ala Arg
       50                  55                  60
   Ser Met Ala Gln Leu Asn His Pro Asn Leu Val Asn Val Tyr Asp Phe
   65                  70                  75                  80
   Ser Ala Thr Asp Gly Leu Val Tyr Leu Val Met Glu Leu Ile Thr Gly
                   85                  90                  95
   Gly Thr Leu Arg Glu Leu Leu Ala Glu Arg Gly Pro Met Pro Pro His
               100                 105                 110
   Ala Ala Val Gly Val Met Arg Gly Val Leu Thr Gly Leu Ala Ala Ala
           115                 120                 125
   His Arg Ala Gly Met Val His Arg Asp Ile Lys Pro Asp Asn Val Leu
       130                 135                 140
   Ile Asn Ser Asp His Gln Val Lys Leu Ser Asp Phe Gly Leu Val Arg
   145                 150                 155                 160
   Ala Ala His Ala Gly Gln Ser Gln Asp Asn Gln Ile Val Gly Thr Val
                   165                 170                 175
   Ala Tyr Leu Ser Pro Glu Gln Val Glu Gly Gly Glu Ile Gly Pro Ala
               180                 185                 190
   Ser Asp Val Tyr Ser Ala Gly Ile Val Leu Phe Glu Leu Leu Thr Gly
           195                 200                 205
   Thr Thr Pro Phe Ser Gly Glu Asp Asp Leu Asp His Ala Tyr Ala Arg
       210                 215                 220
   Leu Thr Glu Val Val Pro Ala Pro Ser Ser Leu Ile Asp Gly Val Pro
   225                 230                 235                 240
   Ser Leu Ile Asp Glu Leu Val Ala Thr Ala Thr Ser Ile Asn Pro Glu
                   245                 250                 255
   Asp Arg Phe Asp Asp Ser Gly Glu Phe Leu Ser Ala Leu Glu Asp Val
               260                 265                 270
   Ala Thr Glu Leu Ser Leu Pro Ala Phe Arg Val Pro Val Pro Val Asn
           275                 280                 285
   Ser Ala Ala Asn Arg Ala Asn Ala Gln Val Pro Asp Ala Gln Pro Thr
       290                 295                 300
   Asp Met Phe Thr Thr His Ile Pro Lys Thr Pro Glu Pro Asp His Thr
   305                 310                 315                 320
   Ala Ile Ile Pro Val Ala Ser Ala Asn Glu Thr Ser Ile Leu Pro Ala
                   325                 330                 335
   Gln Asn Met Ala Gln Asn Met Ala Gln Asn Pro Leu Gln Pro Pro Glu
               340                 345                 350
   Pro Asp Phe Ala Pro Glu Pro Pro Pro Asp Thr Ala Leu Asn Ile Gln
           355                 360                 365
   Asp Gln Glu Leu Ala Arg Ala Asp Glu Pro Glu Ile Asn Thr Val Ser
       370                 375                 380
   Asn Arg Ser Lys Leu Lys Leu Thr Leu Trp Ser Ile Phe Val Val Ala
   385                 390                 395                 400
   Val Ile Ala Ala Val Ala Val Gly Gly Trp Trp Phe Gly Ser Gly Arg
                   405                 410                 415
   Tyr Gly Glu Ile Pro Gln Val Leu Gly Met Asp Glu Val Gln Ala Val
               420                 425                 430
   Ala Val Val Glu Glu Ala Gly Phe Val Ala Val Ala Glu Pro Gln Tyr
           435                 440                 445
   Asp Asn Glu Val Pro Thr Gly Ser Ile Ile Gly Thr Glu Pro Ser Phe
       450                 455                 460
   Gly Glu Arg Leu Pro Arg Gly Glu Asp Val Ser Val Leu Val Ser Gln
   465                 470                 475                 480
   Gly Arg Pro Val Val Pro Asp Leu Ser Glu Asp Arg Ser Leu Ser Thr
                   485                 490                 495
   Val Arg Glu Glu Leu Glu Gln Arg Thr Phe Val Trp Val Asp Gly Pro
               500                 505                 510
   Gly Glu Tyr Ser Asp Asp Val Pro Glu Gly Gln Val Val Ser Phe Thr
           515                 520                 525
   Pro Ser Ser Gly Thr Gln Leu Asp Val Gly Glu Thr Val Gln Ile His
       530                 535                 540
   Leu Ser Arg Gly Pro Ala Pro Val Glu Ile Pro Asp Val Ser Gly Met
   545                 550                 555                 560
   Gly Val Asp Gln Ala Thr Arg Val Leu Glu Arg Ala Gly Leu Ser Val
                   565                 570                 575
   Glu Arg Thr Glu Glu Gly Phe Asp Ala Glu Thr Pro Asn Gly Asp Val
               580                 585                 590
   Tyr Gly Thr Ser Pro Lys Val Ser Thr Glu Val Lys Arg Gly Thr Ser
           595                 600                 605
   Val Val Leu Gln Val Ser Asn Ala Ile Ser Val Pro Asp Val Val Gly
       610                 615                 620
   Met Thr Lys Asp Glu Ala Thr Ala Ala Leu Ala Glu Glu Gly Leu Val
   625                 630                 635                 640
   Val Ala Ser Thr Ser Ile Ile Pro Gly Glu Ala Ala Ser Ser Ala Asp
                   645                 650                 655
   Ala Val Val Thr Val Glu Pro Glu Ser Gly Ser Arg Val Asp Pro Ala
               660                 665                 670
   His Pro Gln Val Ser Leu Gly Leu Ala Gly Glu Ile Gln Val Pro Ser
           675                 680                 685
   Val Val Gly Arg Lys Val Ser Asp Ala Arg Ser Ile Leu Glu Glu Ala
       690                 695                 700
   Gly Leu Thr Leu Thr Thr Asp Ala Asp Asp Asn Asp Arg Ile Tyr Ser
   705                 710                 715                 720
   Gln Thr Pro Arg Ala Arg Ser Glu Val Ser Val Gly Gly Glu Val Thr
                   725                 730                 735
   Val Arg Ala Phe
               740
<210> 3
   <211> 3341
   <212> DNA
   <213> Corynebacterium glutamicum
<220>
   <221> CDS
   <222> (512)..(2731)
   <223> Allele pknD-1547
<220>
   <221> misc feature
   <222> (2501)
   <223> G-A Transition
<220>
   <221> misc_feature
   <222> (2507)
   <223> G-A Transition
<400> 3
   agaacgccat tgcttgagcg cgtcgcataa cttcacgagc caactggcca tgaagtgcat 60
   cgatggggcg accaggaagg gtctcgtctt cggtaaacag gaacgcgagg atttcctcgt 120
   cgctgaagcc accgtcggca agcaaggcaa taactccagg gatgaaacgg ttggtgtttt 180
   ccttcttggt gctcaggaaa gcttctggaa tgtagcgaat accgtcgcgc cggaccacga 240
   tcaatttgtg ttcattgacc agatccatca ccttggtgac aacaacgccg aggcgctcgg 300
   ctgtctccgg aagggtcagc aatggttcat tgtcgggcag ggcgaaggaa gattcattgt 360
   tggaactcac agtcttaatt tagctggttc gagctctaat ggagaatctt tagggtattt 420
   ctgcgcgtgc cgggaatgaa agcaccttct tgacctttga aaacaggatg tcactaccac 480
   ttttgtgtta ccttccgaca tactggaacg c atg gca aac ttg aag gtc ggt    532
                                     Met Ala Asn Leu Lys Val Gly
                                     1               5
   gac gtt tta gag gac agg tat cgg att gaa act ccg att gcc cgg ggt    580
   Asp Val Leu Glu Asp Arg Tyr Arg Ile Glu Thr Pro Ile Ala Arg Gly
           10                  15                  20
   ggt atg tct acc gtg tac agg tgc ctt gat ctt cgt tta gga cgt tcc    628
   Gly Met Ser Thr Val Tyr Arg Cys Leu Asp Leu Arg Leu Gly Arg Ser
       25                  30                  35
   atg gcg ctt aaa gtc atg gaa gaa gat ttc gtt gat gat ccc att ttc    676
   Met Ala Leu Lys Val Met Glu Glu Asp Phe Val Asp Asp Pro Ile Phe
   40                  45                  50                  55
   cgg cag cgt ttc cgt agg gaa gct cgg tca atg gcg cag cta aat cat    724
   Arg Gln Arg Phe Arg Arg Glu Ala Arg Ser Met Ala Gln Leu Asn His
                   60                  65                  70
   cca aat ttg gtc aat gtg tat gat ttt tcc gct act gac ggt ttg gtg    772
   Pro Asn Leu Val Asn Val Tyr Asp Phe Ser Ala Thr Asp Gly Leu Val
               75                  80                  85
   tat ctg gtg atg gag tta atc act ggt ggc acc ttg cgt gag ttg ctg    820
   Tyr Leu Val Met Glu Leu Ile Thr Gly Gly Thr Leu Arg Glu Leu Leu
           90                  95                  100
   gct gag cgg gga cct atg ccc ccg cat gct gct gtg ggc gtt atg cgt    868
   Ala Glu Arg Gly Pro Met Pro Pro His Ala Ala Val Gly Val Met Arg
       105                 110                 115
   ggg gtg ctc acg ggt ctc gcg gct gcc cac cgg gcg ggc atg gtg cac    916
   Gly Val Leu Thr Gly Leu Ala Ala Ala His Arg Ala Gly Met Val His
   120                 125                 130                 135
   cgg gat atc aag cct gac aac gtg ttg atc aat agt gat cac cag gtg    964
   Arg Asp Ile Lys Pro Asp Asn Val Leu Ile Asn Ser Asp His Gln Val
                   140                 145                 150
   aaa ctg tct gat ttc ggc ttg gtt cga gcg gct cac gcc ggc cag tct    1012
   Lys Leu Ser Asp Phe Gly Leu Val Arg Ala Ala His Ala Gly Gln Ser
               155                 160                 165
   cag gac aat cag att gtg ggc acg gtg gct tat ctt tcc cct gag cag    1060
   Gln Asp Asn Gln Ile Val Gly Thr Val Ala Tyr Leu Ser Pro Glu Gln
           170                 175                 180
   gtt gag ggc ggt gag atc ggg ccg gcc agc gac gtg tat tcg gca ggc    1108
   Val Glu Gly Gly Glu Ile Gly Pro Ala Ser Asp Val Tyr Ser Ala Gly
       185                 190                 195
   att gtg ctc ttt gag ctg ctc aca ggc acc acg cct ttt tcg ggc gag    1156
   Ile Val Leu Phe Glu Leu Leu Thr Gly Thr Thr Pro Phe Ser Gly Glu
   200                 205                 210                 215
   gat gat ctc gac cat gca tac gcc cgc ctt acg gaa gtc gtg ccg gca    1204
   Asp Asp Leu Asp His Ala Tyr Ala Arg Leu Thr Glu Val Val Pro Ala
                   220                 225                 230
   ccg agt tcg ctt atc gac ggc gtc ccc tcc ctc atc gat gag ctt gtc    1252
   Pro Ser Ser Leu Ile Asp Gly Val Pro Ser Leu Ile Asp Glu Leu Val
               235                 240                 245
   gcg aca gct acc tcc att aat cct gag gat cgt ttc gat gat tct gga    1300
   Ala Thr Ala Thr Ser Ile Asn Pro Glu Asp Arg Phe Asp Asp Ser Gly
           250                 255                 260
   gag ttt ttg tcc gca ctg gaa gat gtc gca aca gag ttg agc ttg ccg    1348
   Glu Phe Leu Ser Ala Leu Glu Asp Val Ala Thr Glu Leu Ser Leu Pro
       265                 270                 275
   gct ttc cgg gtc cct gtg ccg gtt aat tcc gca gcc aat agg gct aat    1396
   Ala Phe Arg Val Pro Val Pro Val Asn Ser Ala Ala Asn Arg Ala Asn
   280                 285                 290                 295
   gcc cag gtc ccg gat gct cag cca act gat atg ttt acc acc cat atc    1444
   Ala Gln Val Pro Asp Ala Gln Pro Thr Asp Met Phe Thr Thr His Ile
                   300                 305                 310
   ccc aag act cct gag cct gat cac act gcg atc att ccg gtg gcc tca    1492
   Pro Lys Thr Pro Glu Pro Asp His Thr Ala Ile Ile Pro Val Ala Ser
               315                 320                 325
   gca aat gag acg tcg att ctg cct gcg caa aac atg gca caa aat atg    1540
   Ala Asn Glu Thr Ser Ile Leu Pro Ala Gln Asn Met Ala Gln Asn Met
           330                 335                 340
   gcg cag aat ccg ctg caa cct ccg gaa cct gat ttc gcc ccg gag cca    1588
   Ala Gln Asn Pro Leu Gln Pro Pro Glu Pro Asp Phe Ala Pro Glu Pro
       345                 350                 355
   cct ccg gac aca gcg ctg aat att caa gat caa gag ctt gcg cgc gcc    1636
   Pro Pro Asp Thr Ala Leu Asn Ile Gln Asp Gln Glu Leu Ala Arg Ala
   360                 365                 370                 375
   gat gag cca gaa att aat acc gtc agc aat cgt tcc aaa ttg aag ctg    1684
   Asp Glu Pro Glu Ile Asn Thr Val Ser Asn Arg Ser Lys Leu Lys Leu
                   380                 385                 390
   acg ttg tgg tca att ttc gtg gtc gca gtg atc gct gct gtt gct gtt    1732
   Thr Leu Trp Ser Ile Phe Val Val Ala Val Ile Ala Ala Val Ala Val
               395                 400                 405
   ggc ggt tgg tgg ttc ggt tca ggc cgt tac ggt gag att ccg cag gtg      1780
   Gly Gly Trp Trp Phe Gly Ser Gly Arg Tyr Gly Glu Ile Pro Gln Val
           410                     415                     420
   ttg ggc atg gat gag gtc cag gca gta gct gtt gta gag gaa gct ggt      1828
   Leu Gly Met Asp Glu Val Gln Ala Val Ala Val Val Glu Glu Ala Gly
       425                     430                     435
   ttc gtg gca gtg gct gaa cct cag tat gac aat gag gtt ccc act ggt      1876
   Phe Val Ala Val Ala Glu Pro Gln Tyr Asp Asn Glu Val Pro Thr Gly
   440                     445                     450                     455
   tcg att att ggg act gaa cct tct ttt ggt gag cgc ctt cct cgc ggc      1924
   Ser Ile Ile Gly Thr Glu Pro Ser Phe Gly Glu Arg Leu Pro Arg Gly
                   460                     465                     470
   gag gat gtt tct gtc ctc gtc tct caa ggg cgt ccc gtg gtg ccg gat      1972
   Glu Asp Val Ser Val Leu Val Ser Gln Gly Arg Pro Val Val Pro Asp
               475                     480                     485
   ctt agc gag gat cga tcc tta agc acc gtt cgt gaa gag ttg gaa cag      2020
   Leu Ser Glu Asp Arg Ser Leu Ser Thr Val Arg Glu Glu Leu Glu Gln
           490                     495                     500
   cgc acg ttc gtc tgg gtt gat ggc cca ggt gaa tat tct gac gat gtt      2068
   Arg Thr Phe Val Trp Val Asp Gly Pro Gly Glu Tyr Ser Asp Asp Val
       505                     510                     515
   cca gaa gga caa gta gtt tct ttt aca ccg tcg tca ggc acg cag ctt      2116
   Pro Glu Gly Gln Val Val Ser Phe Thr Pro Ser Ser Gly Thr Gln Leu
   520                     525                     530                     535
   gat gtt ggt gaa acc gtg cag atc cat ttg agc cga ggc ccc gcc ccg      2164
   Asp Val Gly Glu Thr Val Gln Ile His Leu Ser Arg Gly Pro Ala Pro
                   540                     545                     550
   gtt gag att cct gat gtc tct ggc atg gga gtg gat cag gca aca cgt      2212
   Val Glu Ile Pro Asp Val Ser Gly Met Gly Val Asp Gln Ala Thr Arg
               555                     560                     565
   gtg ttg gag cgc gca ggt ttg agc gtc gag cgt act gaa gaa ggc ttt      2260
   Val Leu Glu Arg Ala Gly Leu Ser Val Glu Arg Thr Glu Glu Gly Phe
           570                     575                     580
   gat gct gag aca cca aat ggt gat gtc tac ggg act tcg ccc aag gta      2308
   Asp Ala Glu Thr Pro Asn Gly Asp Val Tyr Gly Thr Ser Pro Lys Val
       585                     590                     595
   tct act gag gtc aag cgc gga acc tct gtt gtg ctg cag gtg tcc aat      2356
   Ser Thr Glu Val Lys Arg Gly Thr Ser Val Val Leu Gln Val Ser Asn
   600                     605                     610                     615
   gct att tcg gta ccg gat gtg gtg ggt atg acc aag gac gaa gcc acc      2404
   Ala Ile Ser Val Pro Asp Val Val Gly Met Thr Lys Asp Glu Ala Thr
                   620                     625                     630
   gcg gcg ctt gcg gaa gaa gga ttg gtc gtg gcg tcg aca agc att att      2452
   Ala Ala Leu Ala Glu Glu Gly Leu Val Val Ala Ser Thr Ser Ile Ile
               635                     640                     645
   cct ggt gag gcg gcg agc tcc gct gac gcc gtc gtg acc gtc gag cct     2500
   Pro Gly Glu Ala Ala Ser Ser Ala Asp Ala Val Val Thr Val Glu Pro
           650                 655                 660
   aaa tcc agc agc cgc gtt gat cca gcg cat ccg cag gtc agc ctc ggg     2548
   Lys Ser Ser Ser Arg Val Asp Pro Ala His Pro Gln Val Ser Leu Gly
       665                 670                 675
   tta gct ggg gag att caa gtt cca agc gtg gtt gga cgt aag gtt agc     2596
   Leu Ala Gly Glu Ile Gln Val Pro Ser Val Val Gly Arg Lys Val Ser
   680                 685                 690                 695
   gat gct cga agc att ctg gaa gaa gcc ggt tta acg ctg aca act gat     2644
   Asp Ala Arg Ser Ile Leu Glu Glu Ala Gly Leu Thr Leu Thr Thr Asp
                   700                 705                 710
   gcg gac gac aac gat cga att tat agt caa acc cct cgt gca cgc agc     2692
   Ala Asp Asp Asn Asp Arg Ile Tyr Ser Gln Thr Pro Arg Ala Arg Ser
               715                 720                 725
   gaa gtc tcg gta ggg gga gaa gtt aca gta agg gcg ttt tagtggttcc      2741
   Glu Val Ser Val Gly Gly Glu Val Thr Val Arg Ala Phe
           730                 735                 740
   ctcgttgcag caatggcgaa aacctgctct catcctggcc attttgacgg tgctaggcgt 2801
   actcctgacc cattggttcg cctggccact cacctggccg ctggggctgc gtcttcccgt 2861
   tgatgtagag gtgtactggc agggtgcgcg cgagttttgg ctcgccgatg atctctacga 2921
   catcaggtat gacaccactt tcgacaactt gccgttcacc tatccccctt tcggtgcgtt 2981
   ggtgttcacc ccattgtggt ggattcatga cctctttggt cttctcgtca ccgaacgtgt 3041
   cttcgcgcta atcacgctgc tcaccaccta cgctgtggca gttttcctgc tccgcctggc 3101
   cggcgtgcgc gatcgtgtgt gggaattcgt cgcattcgca gccctgctcg tgtccgcgcc 3161
   ggtgtatttc acactcaata ttgggcaaat aaacgtcatg ctcatggctt taacgctttt 3221
   cgacgtcgcc ctcccccgca gcacgcgcca ttcaggcgtg ctcaaatacg tgccactcgg 3281
   cgtactcacc ggcattgccg ctgcgatcaa actaacccca ctagtgttcg ggctgtattt 3341
<210> 4
   <211> 740
   <212> PRT
   <213> Corynebacterium glutamicum
<400> 4
   Met Ala Asn Leu Lys Val Gly Asp Val Leu Glu Asp Arg Tyr Arg Ile
   1               5                   10                  15
   Glu Thr Pro Ile Ala Arg Gly Gly Met Ser Thr Val Tyr Arg Cys Leu
               20                  25                  30
   Asp Leu Arg Leu Gly Arg Ser Met Ala Leu Lys Val Met Glu Glu Asp
           35                  40                  45
   Phe Val Asp Asp Pro Ile Phe Arg Gln Arg Phe Arg Arg Glu Ala Arg
   50 55 60
   Ser Met Ala Gln Leu Asn His Pro Asn Leu Val Asn Val Tyr Asp Phe
   65 70 75 80
   Ser Ala Thr Asp Gly Leu Val Tyr Leu Val Met Glu Leu Ile Thr Gly
   85 90 95
   Gly Thr Leu Arg Glu Leu Leu Ala Glu Arg Gly Pro Met Pro Pro His
   100 105 110
   Ala Ala Val Gly Val Met Arg Gly Val Leu Thr Gly Leu Ala Ala Ala
   115 120 125
   His Arg Ala Gly Met Val His Arg Asp Ile Lys Pro Asp Asn Val Leu
   130 135 140
   Ile Asn Ser Asp His Gln Val Lys Leu Ser Asp Phe Gly Leu Val Arg
   145 150 155 160
   Ala Ala His Ala Gly Gln Ser Gln Asp Asn Gln Ile Val Gly Thr Val
   165 170 175
   Ala Tyr Leu Ser Pro Glu Gln Val Glu Gly Gly Glu Ile Gly Pro Ala
   180 185 190
   Ser Asp Val Tyr Ser Ala Gly Ile Val Leu Phe Glu Leu Leu Thr Gly
   195 200 205
   Thr Thr Pro Phe Ser Gly Glu Asp Asp Leu Asp His Ala Tyr Ala Arg
   210 215 220
   Leu Thr Glu Val Val Pro Ala Pro Ser Ser Leu Ile Asp Gly Val Pro
   225 230 235 240
   Ser Leu Ile Asp Glu Leu Val Ala Thr Ala Thr Ser Ile Asn Pro Glu
   245 250 255
   Asp Arg Phe Asp Asp Ser Gly Glu Phe Leu Ser Ala Leu Glu Asp Val
   260 265 270
   Ala Thr Glu Leu Ser Leu Pro Ala Phe Arg Val Pro Val Pro Val Asn
   275 280 285
   Ser Ala Ala Asn Arg Ala Asn Ala Gln Val Pro Asp Ala Gln Pro Thr
   290 295 300
   Asp Met Phe Thr Thr His Ile Pro Lys Thr Pro Glu Pro Asp His Thr
   305 310 315 320
   Ala Ile Ile Pro Val Ala Ser Ala Asn Glu Thr Ser Ile Leu Pro Ala
   325 330 335
   Gln Asn Met Ala Gln Asn Met Ala Gln Asn Pro Leu Gln Pro Pro Glu
   340 345 350
   Pro Asp Phe Ala Pro Glu Pro Pro Pro Asp Thr Ala Leu Asn Ile Gln
   355 360 365
   Asp Gln Glu Leu Ala Arg Ala Asp Glu Pro Glu Ile Asn Thr Val Ser
       370                 375                 380
   Asn Arg Ser Lys Leu Lys Leu Thr Leu Trp Ser Ile Phe Val Val Ala
   385                 390                 395                 400
   Val Ile Ala Ala Val Ala Val Gly Gly Trp Trp Phe Gly Ser Gly Arg
                   405                 410                 415
   Tyr Gly Glu Ile Pro Gln Val Leu Gly Met Asp Glu Val Gln Ala Val
               420                 425                 430
   Ala Val Val Glu Glu Ala Gly Phe Val Ala Val Ala Glu Pro Gln Tyr
           435                 440                 445
   Asp Asn Glu Val Pro Thr Gly Ser Ile Ile Gly Thr Glu Pro Ser Phe
       450                 455                 460
   Gly Glu Arg Leu Pro Arg Gly Glu Asp Val Ser Val Leu Val Ser Gln
   465                 470                 475                 480
   Gly Arg Pro Val Val Pro Asp Leu Ser Glu Asp Arg Ser Leu Ser Thr
                   485                 490                 495
   Val Arg Glu Glu Leu Glu Gln Arg Thr Phe Val Trp Val Asp Gly Pro
               500                 505                 510
   Gly Glu Tyr Ser Asp Asp Val Pro Glu Gly Gln Val Val Ser Phe Thr
           515                 520                 525
   Pro Ser Ser Gly Thr Gln Leu Asp Val Gly Glu Thr Val Gln Ile His
       530                 535                 540
   Leu Ser Arg Gly Pro Ala Pro Val Glu Ile Pro Asp Val Ser Gly Met
   545                 550                 555                 560
   Gly Val Asp Gln Ala Thr Arg Val Leu Glu Arg Ala Gly Leu Ser Val
                   565                 570                 575
   Glu Arg Thr Glu Glu Gly Phe Asp Ala Glu Thr Pro Asn Gly Asp Val
               580                 585                 590
   Tyr Gly Thr Ser Pro Lys Val Ser Thr Glu Val Lys Arg Gly Thr Ser
           595                 600                 605
   Val Val Leu Gln Val Ser Asn Ala Ile Ser Val Pro Asp Val Val Gly
       610                 615                 620
   Met Thr Lys Asp Glu Ala Thr Ala Ala Leu Ala Glu Glu Gly Leu Val
   625                 630                 635                 640
   Val Ala Ser Thr Ser Ile Ile Pro Gly Glu Ala Ala Ser Ser Ala Asp
                   645                 650                 655
   Ala Val Val Thr Val Glu Pro Lys Ser Ser Ser Arg Val Asp Pro Ala
               660                 665                 670
   His Pro Gln Val Ser Leu Gly Leu Ala Gly Glu Ile Gln Val Pro Ser
           675                 680                 685
   Val Val Gly Arg Lys Val Ser Asp Ala Arg Ser Ile Leu Glu Glu Ala
   690 695 700
   Gly Leu Thr Leu Thr Thr Asp Ala Asp Asp Asn Asp Arg Ile Tyr Ser
   705 710 715 720
   Gln Thr Pro Arg Ala Arg Ser Glu Val Ser Val Gly Gly Glu Val Thr
   725 730 735
   Val Arg Ala Phe
   740
<210> 5
   <211> 26
   <212> DNA
   <213> Artificial sequence
<220>
   <223> Description of the artificial sequence: Primer pknD_XL-Al
<400> 5
   tctagacggt tggtggttcg gttcag 26
<210> 6
   <211> 26
   <212> DNA
   <213> Artificial sequence
<220>
   <223> Description of the artificial sequence: Primer pknD_XL-E1
<400> 6
   tctagacgcg gcaatgccgg tgagta 26